# EUROPEAN PATENT SPECIFICATION

(11) **EP 2 081 599 B1**
(45) Date of publication and mention of the grant of the patent: **11.04.2012**
(21) Application number: 07733872.1
(22) Date of filing: 14.06.2007
(51) Int. Cl.: A61K 45/06, A61P 35/00

(54) **COMPOUNDS, KITS AND METHODS FOR CONFERRING CYTOPROTECTION**
VERBINDUNGEN, KITS UND VERFAHREN ZUR VERMITTLUNG VON ZYTOPROTEKTION
COMPOSÉS, KITS ET PROCÉDÉS SERVANT À CONFÉRER UNE CYTOPROTECTION

(30) Priority: 14.06.2006 HU 0600497; 19.06.2006 HU 0600508
(43) Date of publication of application: 29.07.2009
(73) Proprietor: University of Debrecen, 4010 Debrecen (HU)
(72) Inventor: NAGY, László, H-4028 Debrecen (HU); SZATMÁRI, István, H-4032 Debrecen (HU)
(74) Representative: Svingor, Adam
(86) International application number: PCT/HU2007/000055
(87) International publication number: WO 2007/144679

(56) References cited:
- WO-A-00/18234
- WO-A-2004/101776
- ISTVAN SZATMARI ET AL: "Peroxisome proliferator-activated receptor gamma-regulated ABCG2 expression confers cytoprotection to human dendritic cells" JOURNAL OF BIOLOGICAL CHEMISTRY, vol. 281, no. 33, August 2006 (2006-08), pages 23812-23823, XP002468074 ISSN: 0021-9258
- MEIER YVONNE ET AL: "ABCG2, encoding the human, breast cancer resistance protein (BCRP), is transactivated by the peroxisome proliferator-activated receptor-gamma (PPARgamma)" HEPATOLOGY, WILLIAMS AND WILKINS, BALTIMORE, MD, US, vol. 44, no. 4, suppl 1, October 2006 (2006-10), page 595A, XP009095303 ISSN: 0270-9139
- GUTMANN H ET AL: "Distribution of breast cancer resistance protein (BCRP/ABCG2) mRNA expression along the human GI tract" BIOCHEMICAL PHARMACOLOGY, PERGAMON, OXFORD, GB, vol. 70, no. 5, 1 September 2005 (2005-09-01), pages 695-699, XP004996351 ISSN: 0006-2952
- COPLAND J A ET AL: "Novel high-affinity PPARgamma agonist alone and in combination with paclitaxel inhibits human anaplastic thyroid carcinoma tumor growth via p21WAF1/CIP1." ONCOGENE 13 APR 2006, vol. 25, no. 16, 13 April 2006 (2006-04-13), pages 2304-2317, XP002468075 ISSN: 0950-9232
- HUISMAN MAARTEN T ET AL: "MRP2 (ABCC2) transports taxanes and confers paclitaxel resistance and both processes are stimulated by probenecid" INTERNATIONAL JOURNAL OF CANCER, vol. 116, no. 5, September 2005 (2005-09), pages 824-829, XP002468076 ISSN: 0020-7136
- KAWAMATA HITOSHI ET AL: "Differentiation-inducing therapy for solid tumors." CURRENT PHARMACEUTICAL DESIGN 2006, vol. 12, no. 3, January 2006 (2006-01), pages 379-385, XP002468077 ISSN: 1381-6128
- SAITO HIKARU ET AL: "A new strategy of high-speed screening and quantitative structure-activity relationship analysis to evaluate human ATP-binding cassette transporter ABCG2-drug interactions" JOURNAL OF PHARMACOLOGY AND EXPERIMENTAL THERAPEUTICS, vol. 317, no. 3, 17 February 2006 (2006-02-17), pages 1114-1124 URL, XP002468078 online ISSN: 0022-3565
- LIANG Y C ET AL: "Suppression of inducible cyclooxygenase and nitric oxide synthase through activation of peroxisome proliferator-activated receptor-gamma by flavonoids in mouse macrophages" FEBS LETTERS, ELSEVIER, AMSTERDAM, NL, vol. 496, 2001, pages 12-18, XP002331304 ISSN: 0014-5793
- DANG ZHI-CHAO ET AL: "Peroxisome proliferator-activated receptor gamma (PPARgamma ) as a molecular target for the soy phytoestrogen genistein." THE JOURNAL OF BIOLOGICAL CHEMISTRY 10 JAN 2003, vol. 278, no. 2, 10 January 2003 (2003-01-10), pages 962-967, XP002468079 ISSN: 0021-9258
- SHUKLA SUNEET ET AL: "Curcumin is a potent modulator of multidrug resistance-linked ABC drug transporter ABCG2." PROCEEDINGS OF THE AMERICAN ASSOCIATION FOR CANCER RESEARCH ANNUAL MEETING, vol. 47, April 2006 (2006-04), pages 145-146, XP001537848 & 97TH ANNUAL MEETING OF THE AMERICAN-ASSOCIATION-FOR-CANCER-RESEARCH (AACR); WASHINGTON, DC, USA; APRIL 01 -05, 2006 ISSN: 0197-016X
- CHEN ANPING ET AL: "Activation of PPAR{gamma} by curcumin inhibits Moser cell growth and mediates suppression of gene expression of cyclin D1 and EGFR." AMERICAN JOURNAL OF PHYSIOLOGY. GASTROINTESTINAL AND LIVER PHYSIOLOGY MAR 2005, vol. 288, no. 3, March 2005 (2005-03), pages G447-G456, XP002468080 ISSN: 0193-1857
- NAKANISHI T ET AL: "Novel 5' untranslated region variants of BCRP mRNA are differentially expressed in drug-selected cancer cells and in normal human tissues: Implications for drug resistance, tissue-specific expression, and alternative promoter usage" CANCER RESEARCH 15 MAY 2006 UNITED STATES, vol. 66, no. 10, 15 May 2006 (2006-05-15), pages 5007-5011, XP002468717 ISSN: 0008-5472

## Description

The present invention relates to combined treatments by PPARg agonists and cytotoxic drugs transportable by ABCG2, various treatments in the field of neoplastic diseases as well as cell therapy, including autologous cell therapy, as well as kits and composition therefor. An in vitro Method for protecting cells against cytotoxic drugs is also provided.

### BACKGROUND ART

### Peroxisome proliferator-activated receptor (PPAR)

The nuclear peroxisome proliferator-activated receptor (PPAR) is a nuclear hormone receptor family comprising three orphan receptors, PPARg (PPARgamma), PPARd (PPARdelta) and PPARa (PPARalpha) that are involved most probably in lipid uptake of the cells.

PPARg is a transcription factor that is activated by lipids, e.g. poly-unsaturated fatty acids and their metabolites, is known to be essential for fat cell formation, and is involved in regulation of genes of lipid uptake, accumulation and storage (Willson, T. M., Lambert, M. H., and Kliewer, S. A. (2001) Annu Rev Biochem 70, 341-367). It was cloned (WO 99/05161), and was shown to be involved in the regulation of glucose homeostasis and to be linked to systemic insulin action (Willson et al., see above).

PPARg is also part of a network of transcriptional regulators coordinately regulating lipid uptake and cholesterol efflux in macrophages by transcriptionally regulating CD36 and the oxysterol receptor LXRa (Liver X receptor-alpha, LXRalpha).

PPARg ligands have been shown to mediate anti-inflammatory activities in vitro and in vivo (Delerive P. et al (2001), J Endocrinol 169, 453-459; Desreumaux, P. (2001) J Exp Med 193, 827-838.). Very recently it has been reported that treatment of NOD mice with PPARg ligands substantially reduced the development of type I diabetes (Augstein, P. et al. (2003). Biochem Biophys Res Commun 304, 378-384.).

Despite intensive research proposals for possible uses of PPAR ligands are still partly controversial and the mechanism through which these ligands exert their effect has remained poorly understood.

Various PPARg agonists have been suggested for use in the treatment of a number of disease conditions, e.g. in neoplastic diseases (Spiegelman et al., WO9825598; Pershadsingh et al, WO 02/076177; Smith, US 6 294 559, 2001; Evans et al, WO 98/29113) cutaneous disorders (Maignan et al., US 2003/0013734A1; Bernardon et al., US 2003/0134885A1), hyperglycemia and diseases associated with type II diabetes (Acton, WO 04/019869) and autoimmune disorders or other inflammatory conditions (Pershadshingh et al., US 6 028 088, 2000; Pershadshingh et al.,WO 02/076177; Winiski, A, GB 2373725). A review on PPARg and its role in metabolic diseases is provided by Willson et al. (Annu. Rev. Biochem. 70, 341 (2001)). Nagy L. et al. (WO2004 101776 A2) have shown that PPARg activation is useful to manipulate professional antigen presenting cells so that an altered expression pattern of CD1 type I and II molecules may be achieved. Such manipulated cells and PPARg modulators may be useful in the treatment autoimmune diseases, allergies, post-transplant conditions or infectious diseases, or in the treatment of a neoplastic disease, e.g. skin cancer, hematological tumors, colorectal carcinoma.

So far, nevertheless, the prior art appears to be silent about the simultaneous application of PPARg modulators and any cytotoxic or chemotherapeutic drugs either for treatment or for research purposes.

Therefore, though use of PPARg agonists was proposed in the art even in the treatment of neoplastic or tumorous diseases, a person skilled in the art should have considered a combination of such a treatment with chemotherapy disadvantageous.

Moreover, in various cell therapies in which PPARg agonists were used, a selective advantage of the successfully treated thereapeutic cells is desirable. No proposal can be found in the art to achieve such a selective advantage by any cytotoxic drug.

### ATP binding-cassette (ABC) transporter G2 (ABCG2)

The ATP binding-cassette (ABC) transporter G2 (ABCG2) also known as breast cancer resistance protein (BCRP), mitoxantrone resistance protein (MXR) and ATP-Binding Cassette Placenta (ABCP) belongs to a transmembrane protein superfamily that mediates the ATP-dependent translocation of a variety of lipophilic substrates (Allikmets, R. et al. (1998) Gene 215, 111-122, Doyle, L. A. et al. (1998) Proc Natl Acad Sci U S A 95, 15665-15670, Miyake, K. et al. (1999) Cancer Res 59, 8-13). Within the human ABC superfamily, ABCG2 belongs to a group of half-transporters that consist of six transmembrane spanning domains that homodimerize to form the active membrane transporter (Sarkadi, B., Ozvegy-Laczka, C., Nemet, K., and Varadi, A. (2004) FEBS Lett 567, 116-120). The ABCG2 gene is highly expressed in the plasma membrane of several drug-resistant cell lines, where it has been shown to transport antitumour drugs including mitoxantrone, topotecan, daunorubicin and doxorubicin (Miyake, K. et al. (1999) Cancer Res 59, 8-13, Jonker, J. et al. (2000) J Natl Cancer Inst 92, 1651-1656, Kawabata, S. et al. (2001) Biochem Biophys Res Commun 280, 1216-1223). In normal tissues, high level expression of ABCG2 is found in the placenta and small intestine, and this protein was shown to play a role in the protection of the organism against toxic xenobiotics (Maliepaard, M. et al. (2001) Cancer Res 61, 3458-3464). ABCG2 is also abundantly expressed in various stem cells, characterized by an increased Hoechst dye efflux capacity (side population, SP) (Zhou, S. et al. (2001) Nat Med 7, 1028-1034). ABCG2 was shown to reduce the accumulation of toxic heme metabolites, thus the pump's expression is part of cell survival strategy under hypoxic conditions (Krishnamurthy, P. et al. (2004) J Biol Chem 279, 24218-24225). Nemet et al. demonstrated that recombinantly expressed ABCG2 may be useful as a selectable marker in certain somatic mammalian cells as well as for protecting such cells from cytotoxic drugs (WO 03/035685).

A recent report suggested that ABCG2 expression is induced by hypoxia, and this regulation involves the hypoxia- inducible transcription factor complex HIF-1 (Krishnamurthy, P. et al. (2004) J Biol Chem 279, 24218-24225).

Understanding regulation mechanism of ABCG2 expression or overexpression is highly desired as it could provide means for downregulation and thereby reduction of multidrug resistance conferred by ABCG2. Moreover, in cases where cytoprotective effect of ABCG2 is advantageous, control of regulation could facilitate artificial induction of such cytoprotection.

However, in spite of its apparently tight regulation, the molecular details of ABCG2 gene expression control are poorly defined and not well understood. In particular, it was not suggested that ABCG2 expression can be regulated by any a nuclear hormone receptor, e.g. nuclear peroxisome proliferator-activated receptor (PPAR), in particular PPARg.

Despite intensive research both in the field of nuclear hormone receptors and ABC transporters, it has not been suggested in the art that PPARg receptor could directly regulate the expression of an ABC transporter, in particular ABCG2.

The present inventors have unexpectedly identified ABCG2 as a tightly regulated gene by one of the nuclear hormone receptors. It is reported herein that PPARg directly and transcriptionally induces ABCG2 expression in a cell type of the human myeloid lineage, monocyte-derived dendritic cells as well as in macrophages. The present inventors have also identified and characterized the regions of the gene involved in this transcriptional activation.

Though therapeutic applications concerning either PPARg or ABCG2 have been proposed, no therapeutic advantage was seen in selecting or protecting PPARg agonist treated cells by cytotoxic drugs transportable by ABCG2.

It has also been surprisingly found that dendritic cells and other cells activated by PPARg express high levels of functional ABCG2 protein, and thereby gain an enhanced capacity to extrude xenobiotics. These features have important consequences to the survival and drug resistance of the treated PPARg activated cells, e.g. human dendritic cells. Thereby, this kind of cell therapeutic methods can be significantly improved, as the treated cells are either protected against cytotoxic drugs transportable by ABCG2, and/or said cells have a selective advantage over untreated cells.

Thus, based on the finding of the present invention treatment of a patient in need of a PPARg agonist treatment and having an ABCG2 negative tumor the combined treatment by a PPARg agonist and a cytotoxic drug transportable by ABCG2 is advantageous.

Moreover, in a ABCG2 positive tumour wherein ABCG2 overexpression is due to PPARg activation a combined treatment by a PPARg antagonist and a cytotoxic drug transportable by ABCG2 is advisable.

The present invention as well as its therapeutic and research applications are based on these findings.

### BRIEF DESCRIPTION OF THE INVENTION

Thus, the according to an aspect the invention relates to a *combination* of a PPARg (peroxisome proliferator activated receptor gamma) agonist and a chemotherapeutic drug transportable by ABCG2 (ATP binding cassette transporter G2), for use in protecting somatic mammalian cells from the chemotherapeutic drug. A combination may be e.g. in the form of any type of composition of matter, preparation or kit.

According to an embodiment the invention relates to *pharmaceutical kit for use* as above, said kit comprising at least a PPARg agonist or composition thereof, and a chemotherapeutic drug transportable by ABCG2 or a composition thereof.

The invention also relates to *a use of a PPARg agonist and a chemotherapeutic drug* transportable by ABCG2 in the preparation of a pharmaceutical composition or pharmaceutical kit for use in the treatment of a neoplastic disease, wherein the neoplastic cells are PPARg negative cells.

Preferably the disease is a neoplastic disease or an immunological disease.

According to this aspect of the invention the neoplastic cells of the patient under chemotherapy are preferably PPARg negative cells. Furthermore, in this aspect of the invention, if chemotherapy is applied, the neoplastic cells of the patient under chemotherapy are advisably ABCG2 negative cells or cells not overexpressing ABCG2.

In a highly preferred embodiment, the treatment of the neoplastic disease comprises both cell therapy and chemotherapy.

The cell therapy comprises normally isolation of cells from a subject; ligand induced activation or increasing expression of PPARg in the cells; optionally differentiating said cells; if desired, administering to said cells a chemotherapeutic drug transportable by ABCG2 (thereby even selection of the cells may be achieved); introducing the cells to a patient.

In the treatment the combination or kit is applied for according to the present aspect, the cells are preferably antigen presenting cells (APCs) or precursor cells of an APC, preferably monocytes, more preferably dendritic cells (DCs). The treatment comprises usually that a chemotherapeutic drug transportable by ABCG2 is administered to the patient.

The invention also relates to a use of a PPARg agonist in the manufacture of a medicament for use in the treatment of a neoplastic disease, wherein the treatment of the neoplastic disease comprises both cell therapy and chemotherapy by a chemotherapeutic drug transportable by ABCG2 and the neoplastic cells of the patient are PPARg negative cells.

According the a further aspect the invention relates to *cell therapeutic methods.*

The combination or kit can be used in a method for combined treatment of a neoplastic disease of a patient by cell therapy and chemotherapy, wherein the neoplastic cells of the patient are PPARg negative cells, comprising the steps of
- isolating cells wherein expression of ABCG2 is inducible by PPARg or PPARg:RXR heterodimer and preferably the cells are PPARg positive cells, from a subject, wherein it is optionally tested whether expression of ABCG2 is inducible in said cells by PPARg or by PPARg:RXR heterodimer,
- activating or increasing the expression of PPARg and, if desired, RXR in said cells, thereby inducing or enhancing the expression of ABCG2 in said cells,
- optionally selecting the cells by a cytotoxic drug transportable by ABCG2,
- differentiating the cells to immature or mature cells and
- introducing the differentiated cells into a patient
- treating the said patient by a chemotherapeutic drug transportable by ABCG2,
wherein preferably the subject the cells are isolated from is identical with the treated patient.

According to a further embodiment the compositions or kits can be used in a method for cell therapy wherein therapeutic cells are selected before administration, comprising the steps of
- isolating cells wherein expression of ABCG2 is inducible by PPARg or PPARg:RXR heterodimer and preferably the cells are PPARg positive cells, from a subject, wherein it is optionally tested whether expression of ABCG2 is inducible in said cells by PPARg or PPARg:RXR heterodimer,
- activating or increasing the expression of PPARg and, if desired, RXR in said cells, thereby effecting a desired alteration in the phenotype of the cells and inducing or enhancing the expression of ABCG2 in the same cells,
- selecting the cells of altered phenotype and expressing ABCG2 by a cytotoxic drug transportable by ABCG2,
- differentiating the cells to immature or mature cells and
- introducing the differentiated cells into a patient,
wherein preferably the subject the cells are isolated from is identical with the treated patient.

According to this embodiment the disease treated may be e.g. any of the following
- a neoplastic disease, e.g. skin cancer, hematological tumors, colorectal carcinoma,
- an immunological disease, e.g. an autoimmune disease (e.g. type 1 diabetes, multiple sclerosis, autoimmune encephalomielitis, anterior chamber-associated immune deviation (ACAID), lupus erithematosus, autoimmune hepatitis), an allergy, a hypersensitivity reaction, a post transplant condition (thereby helping graft acceptance, e.g. of corneal, cardiac, bone marrow, kidney, liver etc. allografts or xenografts), inflammatory conditions,
- an infectious disease.

According to this aspect optionally a further treatment step may be performed on the isolated cells.

According to this aspect the isolated cells to be treated are preferably antigen presenting cells (APCs) or precursor cells of an APC, preferably monocytes, more preferably dendritic cells (DCs). In a further preferred embodiment the isolated cells are macrophages.

According to this aspect preferably PPARg and, if desired, RXR is activated in said cells by administering to the cells a PPARg agonist and, if desired, an RXR agonist (e.g retinoids or related compounds).

According to a further aspect the invention relates to *methods for protecting cells.*

Thus, the invention relates to an in-vitro process for protecting somatic mammalian cells against the effect of a cytotoxic drug transportable by ABCG2, comprising the steps of
- providing said cells in an isolated form,
- enhancing the expression of ABCG2 by activating or increasing the expression of PPARg and, if desired, RXR in said cells,
- exposing the cells either *in vivo* or *in vitro* to the effect of a cytotoxic drug transportable by ABCG2.

In a preferred embodiment PPARg is activated by administering to the cells a PPARg agonist. Preferably RXR may also be specifically activated by administering to the cells an RXR agonist (e.g. a retinoid drug).

In a further preferred embodiment expression of PPARg and, if desired, of RXR is effected by gene transfer into the therapeutic cells.

The invention also relates to a PPARg agonist for use in conferring cytoprotection for mammalian immune cells against a cytotoxic drug transportable by ABCG2.

According to the present aspect of the invention the cells are preferably antigen presenting cells (APCs) or precursor cells of an APC, preferably monocytes, more preferably dendritic cells (DCs) or macrophages.

The application also discloses isolated nucleic acids comprising at least one PPARg responsive element.

The isolated nucleic acid comprising at least one PPARg responsive element has the nucleotide sequence of any of the following: AGGGCAGAGGGCA (SEQ ID NO 1), TAACCTTTAACCT (SEQ ID NO 2) or TAACATCTGACCT (SEQ ID NO 3), TAACTTTTGACCT (SEQ ID NO 4) or TAACTTTTAACCT (SEQ ID NO 5) or a complementer thereof.

The isolated nucleic acid has at most 150 bp, preferably at most 140 bp, more preferably at most 130 bp, 125 bp, 123 bp, 122 bp or 121 bp length, comprising three PPARg responsive elements.

The isolated nucleic acid comprises at least a 121 bp fragment of SEQ ID NO 6 or a sequence at least 80% identical therewith, said fragment carrying at least one, preferably two, more preferably three PPARg responsive elements.

The isolated nucleic acid has a nucleotide sequence which is in at least 70%, or at least 80% or at least 90% identical with a mammalian genomic sequence corresponding to that of a fragment of the -3946 to -3796 segment of the ABCG2 promoter region counting from the position of the beginning of the 5' longest cDNA sequence, said fragment carrying at least one, preferably two, more preferably three PPARg responsive elements. Preferably, the said mammalian genomic sequence corresponds to any of SEQ ID NO 6, 7 or 9.

In a preferred embodiment any of the three PPARg responsive elements have any of the following sequences: SEQ ID NO 1, 2, 3, 4 or 5 or a mutant sequence different by 2 or 1 nucleotides therefrom or a complementer thereof or of the mutant sequence. Highly preferably, the three PPARg responsive elements have, in the 5' to 3' direction, the following sequences: SEQ ID NO 1, 2, 3, 4 or 5, respectively.

The invention also discloses an isolated nucleic acid capable of hybridizing under stringent conditions to a nucleic acid as defined above.

In a further aspect the present application discloses *vectors* comprising the nucleic acid cited above Preferably, the vector further comprises at least one gene encoding a protein, said gene being operably linked to one or more of the PPARg responsive element(s) as defined above. Preferably, the gene is a gene encoding an active ABCG2 transporter protein. Said gene may also be a reporter gene, or the plasmid may carry both an ABCG2 gene and a reporter gene operably linked.

A method is provided to prepare an artificial transcription repressor (repression factor), according to which such a factor is prepared by methods using the sequences of the nucleic acids disclosed herein, in particular the PPARg responsive elements.

Thus, the application further discloses a recombinant artificial transcription repressor protein capable of binding to at least one of the PPARg responsive elements as defined above. The artificial transcription repressor shall be capable of blocking transcription of a gene operably linked to the PPARg responsive element. This can be easily tested by methods disclosed herein.

The artificial transcription repressor can be a Zn finger protein, wherein preferably said Zn finger protein comprises a Zn finger domain recognizing
- an 18 bp sequence comprising a PPARg responsive element, or
- a 9 bp sequence within a PPARg responsive element.

### BRIEF DESCRIPTION OF THE DRAWINGS

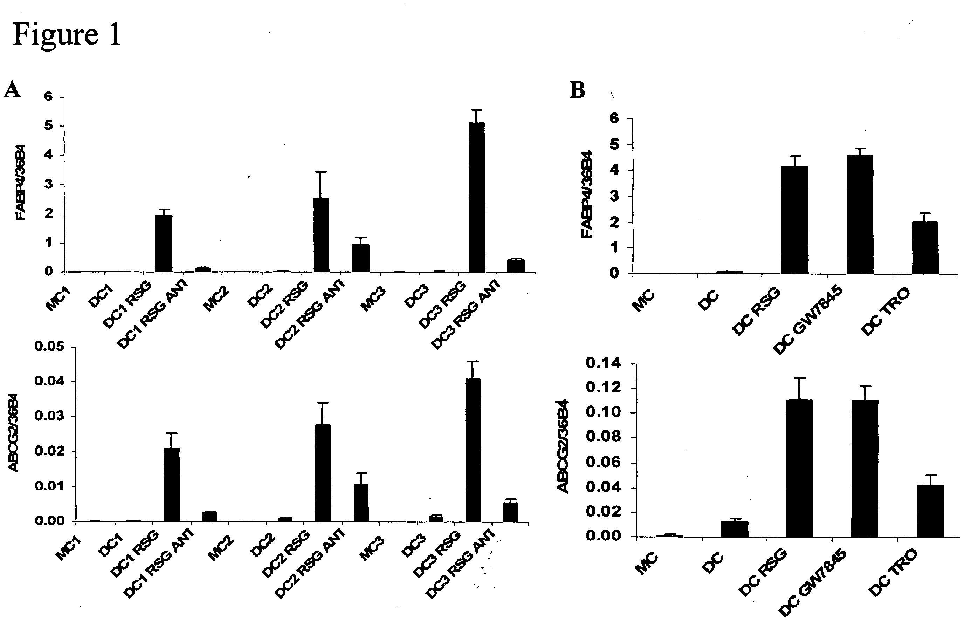

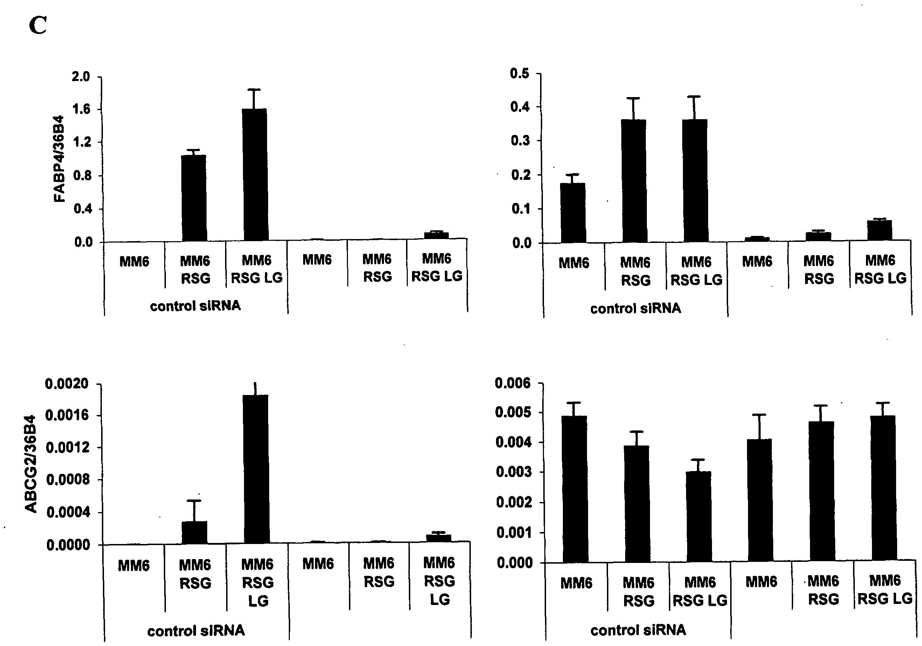

FIGURE 1. **PPARg ligand treatment upregulates the expression of ABCG2.** *A*, FABP4 and ABCG2 transcript is highly upregulated in PPARg ligand (rosiglitazone=RSG) treated DCs. Transcript levels were determined from monocytes (MC), vehicle or PPARg ligand treated DCs (2.5 µM RSG) alone or with a PPARg specific antagonist (ANT): 5 µM GW9662 by RT-Q-PCR. Gene expression data are expressed as a ratio of FABP4 or ABCG2 transcripts relative to 36B4 expression ±SD. Data were obtained from 3 individuals. B, Transcript levels of ABCG2 and FABP4 were determined in DC treated with various ligands: 2.5 µM RSG, 100 nM GW347845X (abbreviated as GW7845), 1 µM troglitazone. C, The expression of FABP4 and ABCG2 is impaired in PPARg knock-down MM6 cells. MM6 cells were electroporated with a control or PPARg specific siRNA as described in Experimental Procedures. After electroporation MM6 cells were cultured for 12 h, thereafter cells were treated with 2.5 µM RSG alone or along with an RXR agonist (100 nM LG268=LG) and cultured for 24 h. Transcript levels of FABP4, ABCG2, PPARg and PPARd were determined by RT-Q-PCR.

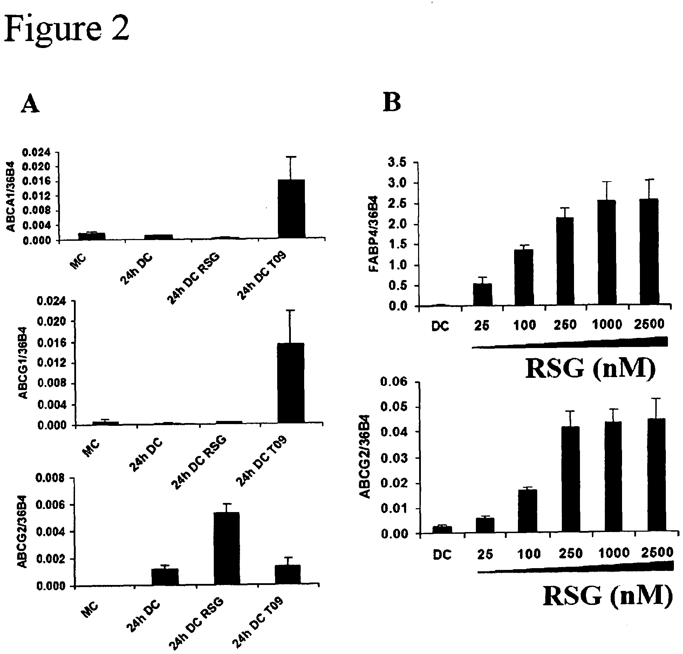

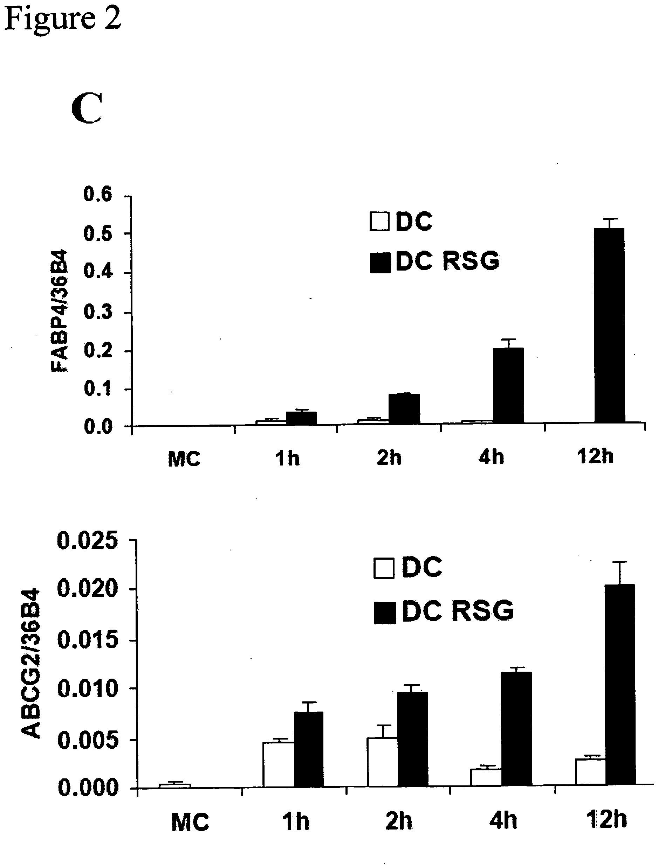

FIGURE 2. **ABCG2 is regulated by PPARg in a mechanism independent of LXR**. *A*, mRNA levels of ABCA1, ABCG1 and ABCG2 were determined with RT-Q-PCR. Cells were cultured for 24 h and treated with 1 µM rosiglitazone (RSG) or with an LXR specific ligand: 1µM T0901317 (T09). *B*, Dose-dependent induction of FABP4 and ABCG2. Cells were treated with the indicated concentration of RSG; the mRNA expression was determined by RT-Q-PCR *C*, Kinetics of FABP4 and ABCG2 induction. Cells were cultured for 12 h, thereafter cells were treated with 1 µM RSG and harvested at the indicated time points and the mRNA expression was determined by RT-Q-PCR.

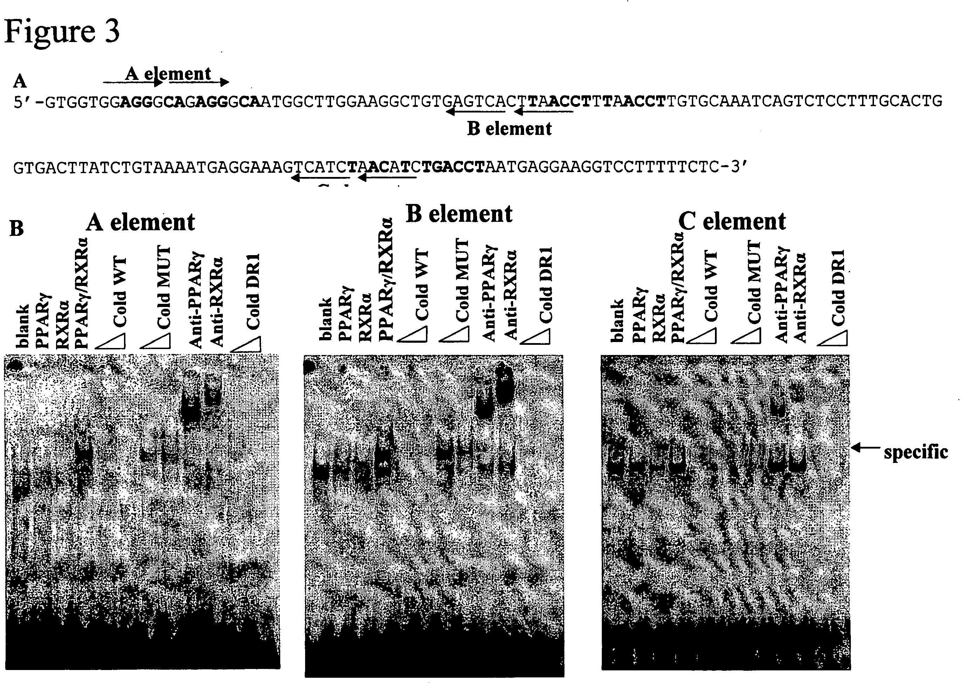

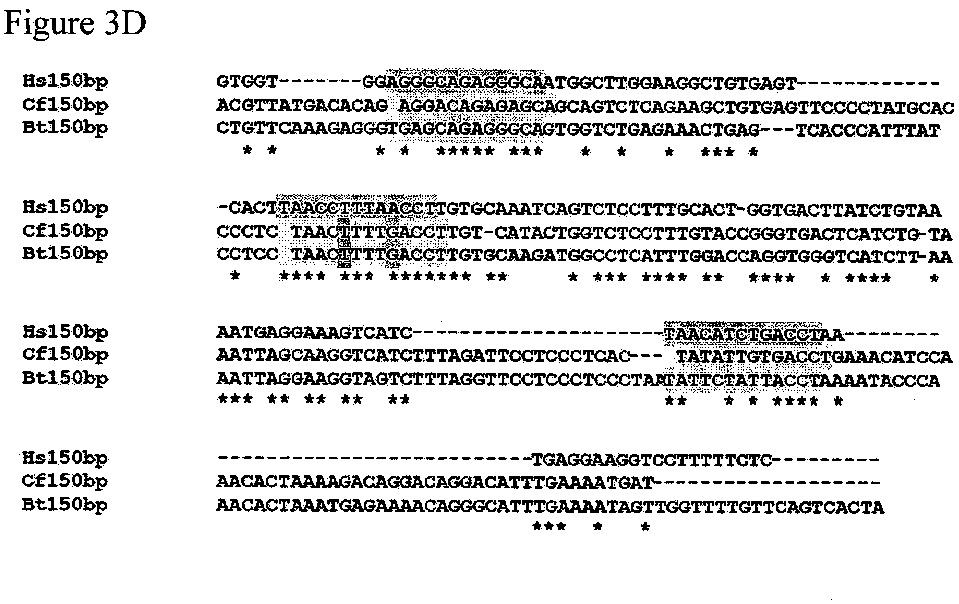

FIGURE 3. **Identification of PPAR:RXR binding sites in the human ABCG2 gene**. *A*, Sequence of a portion of the human ABCG2 upstream region (-3946/-3796 bp), highlighting the three putative PPAR responsive elements (elements A, B and C). *B*, Electrophoretic mobility shift assay (EMSA) was carried out by using in vitro-translated receptors of PPARg, RXRalpha and α³²P-labeled oligonucleotides as described in Experimental Procedures. For competition experiments, cold (cold WT), mutated (cold mut) or consensus DR1 oligonucleotides were used at x10 and x20 concentrations. For supershift experiments, the receptors were preincubated with the indicated antibodies prior to the binding reaction. *D* (SFIGURE 1.) Alignment of human (Hs), dog (Cf) and bovine (Bt) genomic sequences from the ABCG2 upstream region.

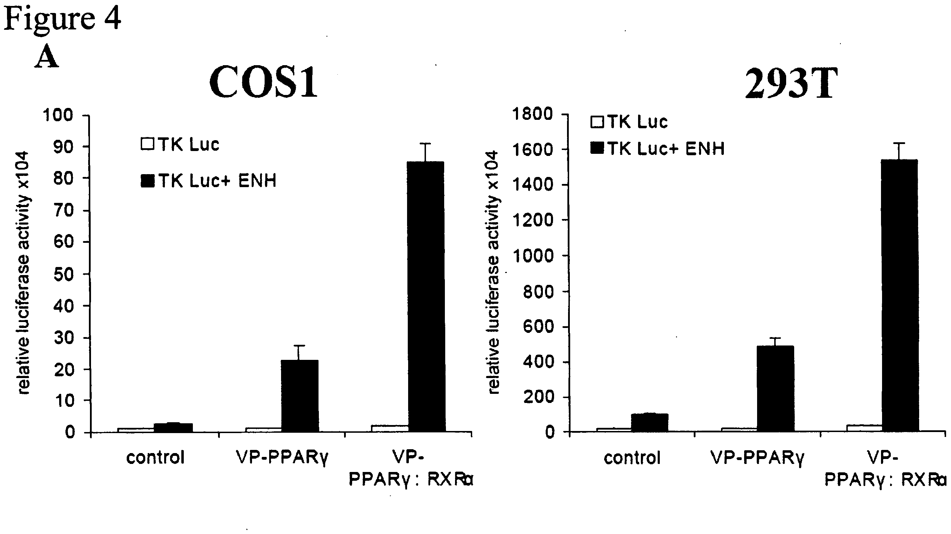

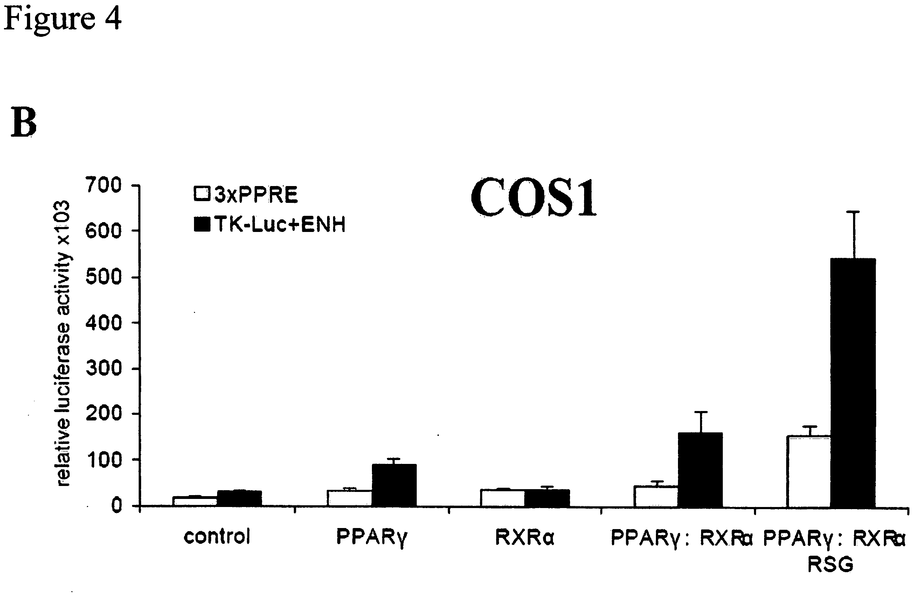

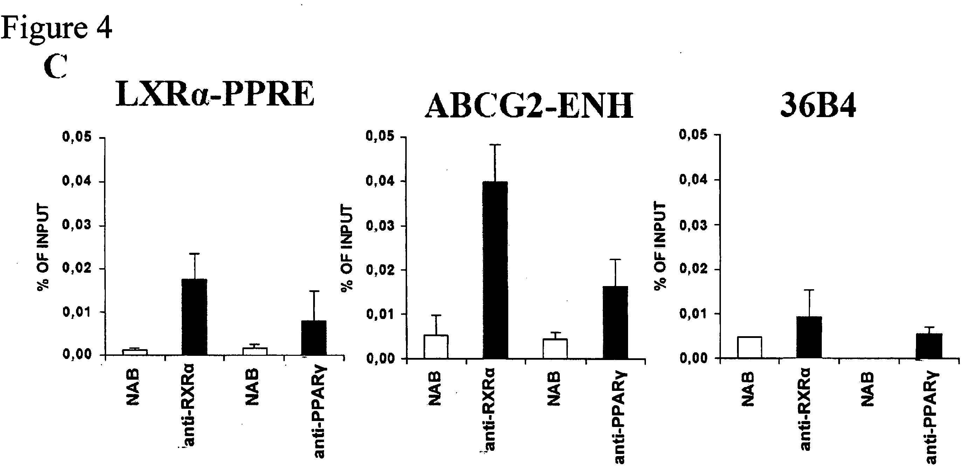

FIGURE 4. **An upstream sequence of the human ABCG2 gene, containing three DR1s, is able to promote transcription in a PPARg dependent manner in a cell based binding assay**. *A*, COS1 or 293T cells were cotransfected with TK-Luc or with TK-Luc enhancer, the VP16 fusion protein of PPARg with or without RXRα, and β-galactosidase. Cells were lysed after 24 h, and the luciferase and β-galactosidase activity were assayed as described in Experimental Procedures. All experiments were done in triplicates, ±SD. *B*, COS1 were cotransfected with the indicated nuclear receptors, TK-Luc enhancer or with luciferase reporter construct containing 3 PPAR responsive elements (3xPPARE), and β-galactosidase. The indicated cells were treated with 1 µM rosiglitazone (RSG). C, Chromatine immunprecipitation (ChIP) was performed with anti-RXR and anti-RAR antibodies using chromatine obtained from PPARg transduced 293T cells. DNA content was analyzed with RT-Q-PCR assays specific for the genomic region of LXRa containing a PPRE (LXR), the identified ABCG2 enhancer region (ABCG2) and a genomic region of 36B4 gene (36B4). The results are shown as percentages of input DNA. All measurements were done in triplicate. As a negative control no-antibody control (NAB) was used.

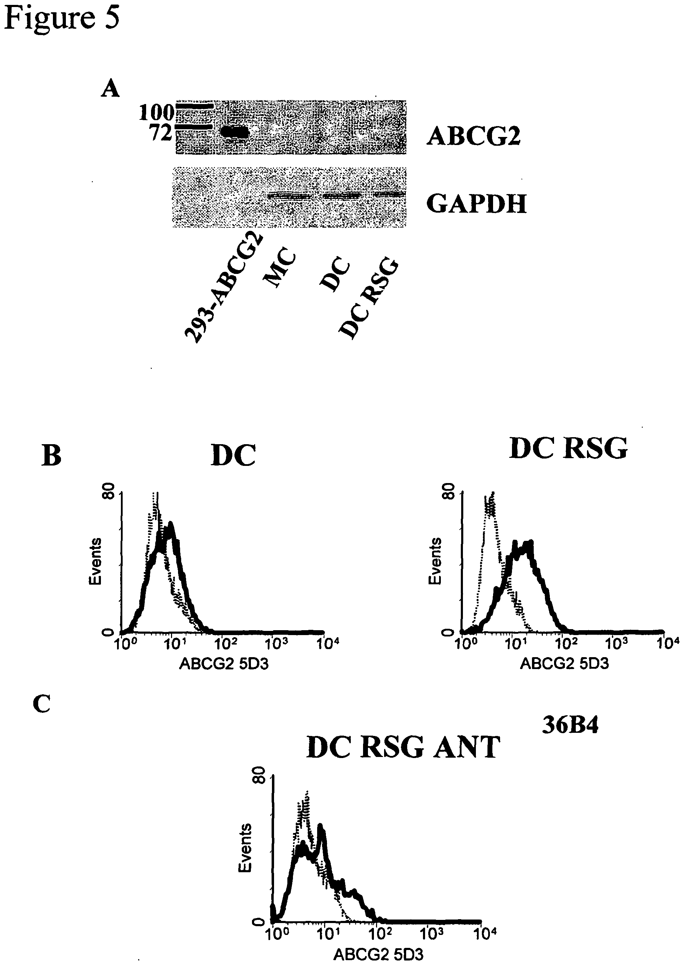

FIGURE 5. **ABCG2 protein is expressed on PPARg instructed DCs**. *A*, Western blot analysis of ABCG2 protein expression in differentiating DC. 50 µg of cell extract from the indicated samples (monocyte-MC, the indicated cells were treated with 2.5 µM RSG) were subjected to Western blot analysis, the identity of the 70 kD bands was confirmed by co-migration with a band seen in the extract (2 µg) of ABCG2 transfected 293T cells. GAPDH was used as a loading control. *B*, Determination of the cell surface expression of ABCG2 in DCs. Control or ligand treated cells were fixed and stained with an unlabeled anti-ABCG2 (5D3) or isotype-matched control and after 30 min incubation with anti mouse FITC-labeled antibody. Cell surface molecule specific antibodies indicated (solid line) *vs*. isotype-matched control (dotted line). Cells were treated with 2.5 µM rosiglitazone (RSG) alone or RSG plus a PPARg antagonist (ANT, GW9662).

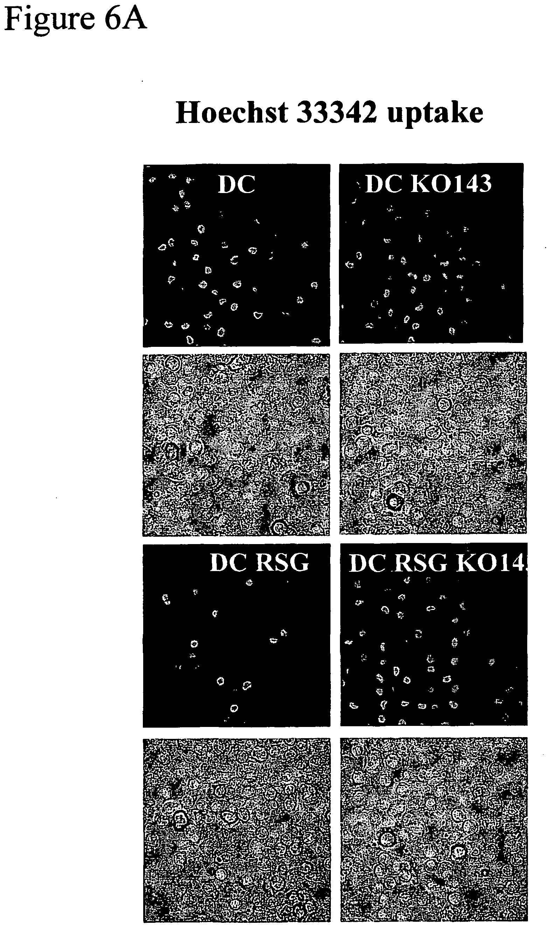

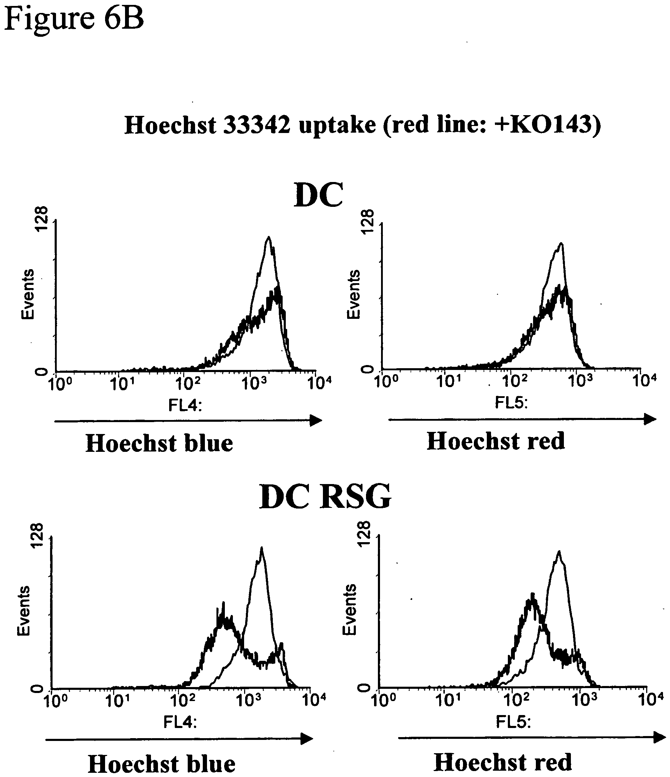

FIGURE 6. **Enhanced efflux of Hoechst 33342 in DCs upon PPARg activation**. *A*, Cells were incubated with 0.5 µg/ml Hoechst dye for 1 h at 37 °C with or without KO143 (1 µM), then subjected to microscopic analysis as described in Experimental Procedures. B, The same samples were quantitatively analyzed with a Becton Dickinson FACSVantage flow cytometer.

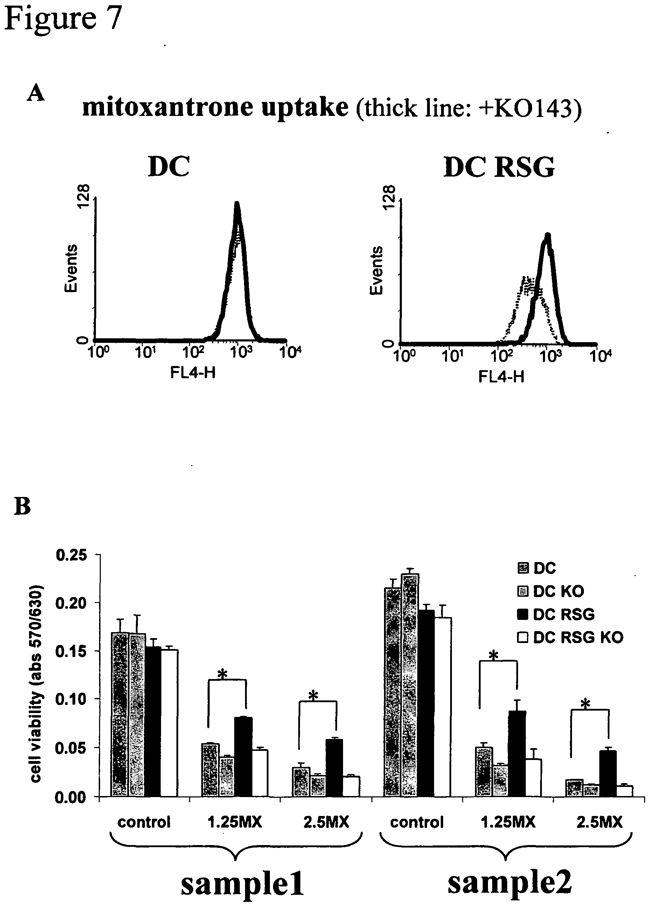

FIGURE 7. **PPARg activated DCs have an enhanced viability upon mitoxantrone exposure**. *A*, Efflux of mitoxantrone by ABCG2 in DCs. Cells were incubated with 20 µM mitoxantrone in the presence or absence of 1 µM ABCG2 inhibitor (KO143) for 30 min and washed with ice-cold PBS and finally the intracellular accumulation of mitoxantrone was assessed by flow cytometry. *B*, Control or PPARg activated (2.5 µM RSG) DCs were cultured for 48 h and treated with 1.25 or with 2.5 µg/ml mitoxantrone. Cell cytotoxicity was assessed with MTT assay as described in Experimental Procedures. **p<0.01, ***p<0.001.

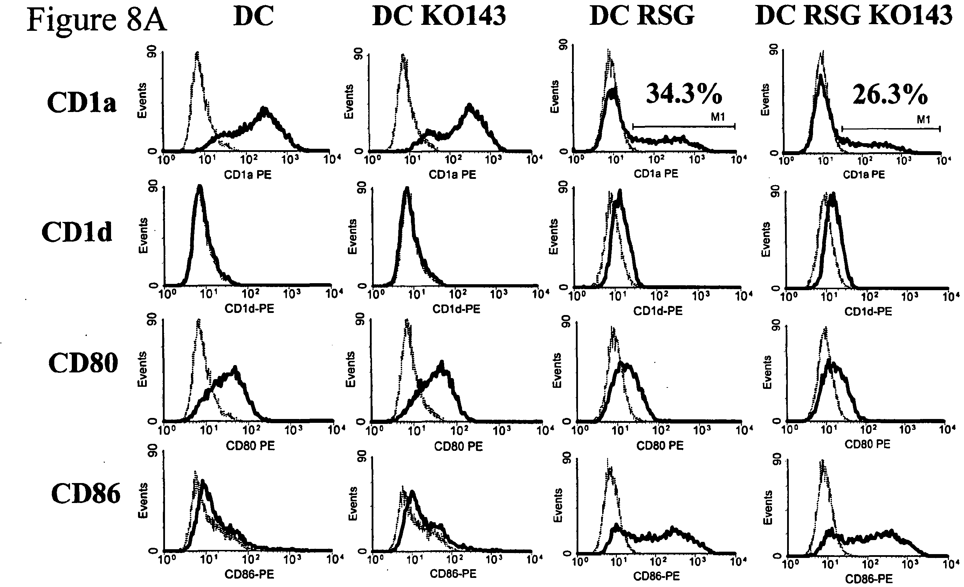

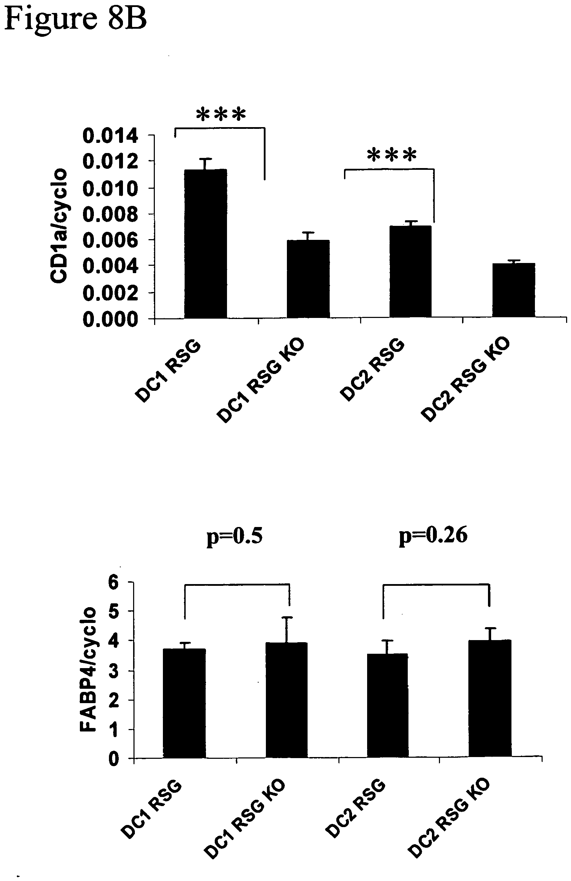

FIGURE 8. **Inhibition of ABCG2 activity slightly modifies expression of CD1a**. *A*, Characterization of CD1a, CD1d, CD80 and CD86 cell-surface expression on DCs treated with ligands: 2.5 µM RSG alone or with 1 µM ABCG2 inhibitor (KO143). Data obtained with specific antibodies indicated (solid line) *vs*. isotype matched control (dotted line). *B*, CD1a and FABP4 transcripts levels were determined from vehicle (DMSO/ethanol) or PPARg ligand treated DCs (2.5 µM RSG) alone or with 1 µM ABCG2 inhibitor (KO143). Gene expression data are expressed as a ratio of CD1a and FABP4 transcripts relative to cyclophilin A (cyclo) expression SD. ***p<0.001.

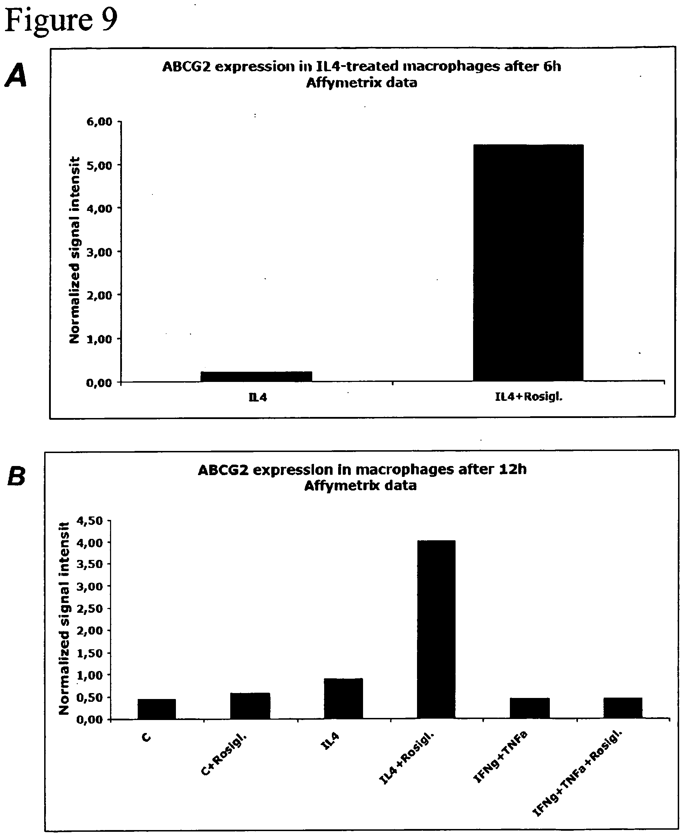

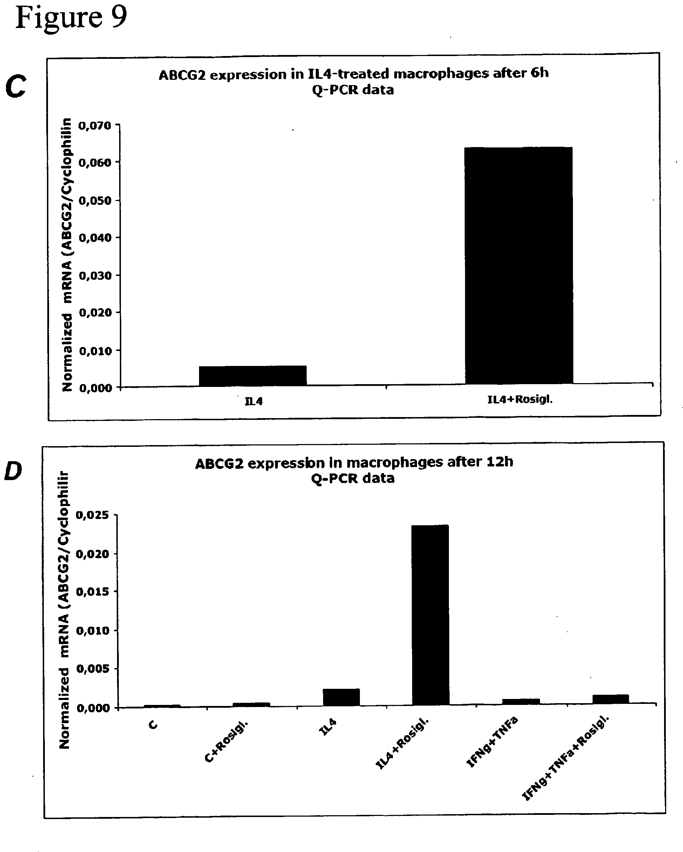

FIGURE 9. **PPARg ligand treatment upregulates the expression of ABCG2 in macrophages**. *A*, *C* ABCG2 expression is highly upregulated in IL4-treated (differentiated) macrophages after 6h PPARg ligand (rosiglitazone=RSG) treatment according to both Affymetrix data and Quantitative PCR. Similar results were obtained after 12 h. Without IL-4 treatment upregulation of ABCG2 was not observed *C*, *D.*

### DEFINITIONS

The term "agonist", as used herein, refers to a molecule which is capable of causing an increase in the biological activity of a biologically active molecule, e.g. a receptor. In general, an increase in activity may be due to any effect, including an alteration the activity of the biologically active molecule, e.g. receptor or an alteration of gene transcription or translation of the receptor. Particularly, an agonist is a drug that has affinity for and stimulates physiologic activity at cell receptors normally stimulated by naturally occurring substances, thus triggering a biochemical response.

As used herein, the term "PPARg agonist" refers to a compound or composition which, when combined with PPARg, directly or indirectly stimulates or increases an in vivo or in vitro reaction typical for the receptor (e.g., transcriptional regulation activity). The present invention contemplates that any known or future identified PPARg agonist will find use with the present invention.

The terms "antagonist" or "inhibitor,", as used herein, relate to a molecule which, when interacting with a biologically active molecule, blocks or negatively regulates the biological activity of the biologically active molecule, e.g. a receptor. Agonists, inhibitors and antagonists can affect the biology of entire cells, thereby organs, or organisms.

An "increase or a decrease" in activity, expression, concentration etc. refers to a change which is detectable under the given conditions, considering the statistical error or the signal/noise ratio of the given measurement.

By "autologous" is meant something (e.g. cells, tissues etc.) derived from the same organism.

The term "isolated" relates to a substance the environment of which has been significantly modified, relative to its natural environment, by Man. Preferably, an isolated substance is substantially or essentially free from certain components that normally accompany it in its natural state.

The term "patient" refers to subjects of mammalian, for example human beings and includes any individual it is desired to examine or treat using the methods of the invention. However, it will be understood that "patient" does not imply that symptoms are present. Suitable animals that fall within the scope of the invention include, but are not restricted to, primates, livestock animals, laboratory test animals, companion animals etc.

### DETAILED DESCRIPTION OF THE INVENTION

Below the invention is disclosed in more detail with reference to certain exemplary embodiments. The scope of the invention is, however, not limited to these exemplary embodiments and any solution, which is characterized by the features of the invention or variant or equivalent thereof, which can be obtained therefrom by obvious modifications, is to be considered as being within the scope of the invention.

The functional and adaptive specification of human DCs is a well established concept (Shortman, K., and Liu, Y. J. (2002) Nat Rev Immunol 2, 151-161; Dubsky, P. et al. (2005) J Clin Immunol 25, 551-572). However the regulatory processes and the molecular underpinnings of functional plasticity or subtype specifications is largely unknown. Previously we and other have provided evidence that nuclear hormone receptors contribute to these processes (Szatmari, I. et al. (2004) Immunity 21, 95-106; Gosset, P. et al. (2001) Eur J Immunol 31, 2857-2865; Nencioni, A. et al. (2002) J Immunol 169, 1228-1235).

The present inventors demonstrated herein the up-regulation of the transporter both at the mRNA and the protein level, the proper cell surface localization as well as the xenobiotic transport activity of the pump. Moreover, a specific enhancer region containing PPAREs, preferably three PPAREs, directly binding PPARg, in the upstream region of the ABCG2 gene has been identified.

It is known that certain ABC transporters are regulated by nuclear hormone receptors. It was described that the increased expression of both ABCA1 and ABCG1 occurs via the oxysterol-activated nuclear receptor, the liver X receptor (LXR), in macrophages (Repa, J. J. et al (2000) Science 289, 1524-1529). In addition, in this cell type, ABCA1 expression is also induced by PPARg activators, through the activation of LXRa receptor (Chawla, A. et al. (2001) Nat Med 7, 48-52, Chawla, A. et al. (2001) Mol Cell 7, 161-171). However, regulation of ABCG2 via PPARg has not been even suggested in the art.

### Abrogation of PPARg dependent ABCG2 drug resistance

It has now been found by the present inventors that ABCG2 induction was receptor and subtype specific. Neither LXR ligands nor retinoids (data not shown), regulators of other transporters, induced the expression of this pump. When cells were treated with PPARa or PPARd ligands poor responses were obtained (data not shown). Furthermore, ABCG2 induction was abrogated by the addition of a PPARg specific antagonist and by using siRNA against PPARg. Importantly, the PPARg-dependent regulation appears to be direct. Three PPAR response elements (direct repeat 1=DR1) were identified and characterized in the upstream. region of the human ABCG2 gene. Gelshift and transient transfection analyses as well as chromatin immunoprecipitation confirmed the specific binding of PPARg:RXR heterodimer to this enhancer region. This upstream enhancer allows novel insights into the promoter/enhancer structure and regulation of ABCG2 as well. Earlier studies identified several Spl sites in the -222bp to -49bp promoter region of ABCG2, a CCAAT-box is also present in the -274bp position, and CpG islands are found in its downstream proximity (Bailey-Dell, K. J. et al. (2001) Biochim Biophys Acta 1520, 234-241). In addition, a novel Estrogen Response Element (ERE) has been identified in the -188bp to -172bp segment, which triggers an enhanced transcription of human ABCG2 in ER-positive cells (Ee, P. L., et al. (2004) Cancer Res 64, 1247-1251).

Thus, the present data indicate the presence of a relatively distant (-3946/-3796bp) major regulatory site, responsible for PPARg:RXR regulation of this gene.

It is an intriguing question whether this regulation is a DC and/or at least myeloid cell type specific phenomenon, or ABCG2 can be induced in other PPARg positive cell types as well. It is shown herein that the PPARg dependent up-regulation of ABCG2 is not restricted to DCs. This mechanism was found to be valid in normal macrophages (Fig. 9).

Moreover, the mechanism has been confirmed in the monocyte/macrophage cell line, MM6. The data presented herein show that in PPARg receptor positive tumor cells activation of this receptor causes the appearance of a multidrug resistance phenotype, due to ABCG2 over-expression.

The above findings allow abrogation or alleviation of multidrug resistance during chemotherapy of cancers, wherein multidrug resistance has been evolved by this regulatory mechanism. More closely, expression of ABCG2 can be blocked or significantly reduced by blocking the PPARg responsive elements with a transcription repressor. The preparation of such transcription repressors targeting a given sequence is known in the art. For example the preparation of artificial (designer) Zn-finger proteins is described and reviews in several pieces of the art [see e.g. Roger R. et al. Nature Biotechnology 20, 135 - 141 (2002); Falke D. et al. Nucleic Acids Research, 2003, Vol. 31, No. 3 e10; Yaghmai R et Cutting GR, Mol Ther. 2002 Jun;5(6):685-94; Papworth M et al., Gene. 2006 Jan 17;366(1):27-38. Ren D et al., Genes Dev. 2002 Jan 1;16(1):27-32.]. Besides Zn-finger proteins, a number of other types of trascription regulator protein is known, including transcription repressors, such as leucine zippers. In an alternative embodiment, binding domain of receptors can be utilized, e.g. the binding domain of PPARg or the PPARg:RXR complex.

As shown above, the same result in these malignancies may be achieved by antagonizing the PPARg protein by a known antagonist. Such antagonists are well-known in the art and disclosed e.g. in WO 2004/101776 and references cited therein.

Translation of PPARg can be abrogated by an siRNA as well as by an antisense RNA as well. These technologies are well-known in the art.

If the action of PPARg is abrogated, the enhancement in ABCG2 expression does not occur, which results in the amelioration of multidrug resistance. This allows chemotherapy, or reduction of necessary doses in chemotherapy by a drug transportable by ABCG2.

### Resistance and cytoprotection conferred by PPARg dependent ABCG2 induction

The expression of ABCG2 is clearly associated with the modulation of xenobiotic absorption, distribution, and toxicity in many different tissues. The wide substrate specificity and tissue distribution of this active transporter thus plays a major role in pharmacotherapy. In hematopoietic stem cells the side population (SP) phenotype, based on Hoechst 33342 efflux, is provided by a high-level ABCG2 expression. More recently, pheophorbide A, PPIX and heme have all been shown to be ABCG2 substrates, indicating a role for this protein in the cytoprotection of stem cells and erythrocytes (Zhou, S. et al. (2004) Blood 105(6):2571-6; Jonker, J. W. et al. (2002) Proc Natl Acad Sci U S A 99, 15649-15654) Our experiments fully support this role in dendritic cells by showing that PPARg activated DCs accumulated less Hoechst dye and mitoxantrone. This effect was reverted by a specific and selective ABCG2 inhibitor, indicating that the effect is ABCG2 dependent. Consistent with the enhanced mitoxantrone efflux, we also found that PPARg treated DCs showed an increased resistance to the anti-neoplastic and immunosuppresive drug, mitoxantrone. Again, this effect was due to the function of enhanced ABCG2 expression and activity. Therefore this pathway, if activated, arms the DCs with the capacity to withstand xenobiotic and toxic noxa. Interestingly, it has been shown that PPARg ligand treatment induced the expression of an UDP-glucuronosyltransferase (UGT1A9) in human hepatocytes and macrophages (Barbier, O. et al. (2003) J Biol Chem 278, 13975-13983). UGTs catalyze the glucuronidation reaction, which is a major pathway in the catabolism and elimination of numerous xenobiotics. It follows from these results that PPARg has the potential to coordinately regulate both the inactivation and elimination of xenobiotics and thus protects these cells.

Thus, protection against cytotoxic drugs transportable by ABCG2 can be elicited in any cell in which the herein described mechanism is valid. This can be utilized both in research, first of all cancer research as well as treatment. In particular, cell transplantation methods by manipulated professional APCs (antigen presenting cells) are described WO2004/101776, wherein the cells are treated by PPARg agonists thereby altering their phenotype. The present invention provides an additional advantage of selecting the cells treated by the agonists. In fact, PPARg agonist are used for treatment in a number of deseases as described e.g. in the "Background art" section of the present application. In cases wherein the mechanism described herein is active, the cells affected by the PPARg agonist are protected by the appropriate cytotoxic drug. As mentioned above, this phenomenon can be utilized in selecting the treated cells in a cell therapy, e.g. an autologous cell therapy.

PPAR g agonists are well known in the art and described e.g. in US6436993. Such compound include e.g. troglitazone, WY14,643, GW0072, rosiglitazone (BRL 49653), L-764406, 15-deoxy-Delta12, 14-prostaglandin J2 (15d-PGJ2) and oxidized linoleic acid (9- and 13-HODE), (2S)-((2-benzoylphenyl)amino)-3-4-[2-(5-methyl-2-phenyloxazol-4-yl)ethoxy]phenylpropanoic acid, 2(S)-((2-benzoylphenyl)amino)-3,4-[2-(5-methyl-2-pyridin-4-yloxazol-4-yl)ethoxy]phenylpropionic acid, 2(S)-((2-benzoylphenyl)amino)-3-(4-2-[5-methyl-2-(4-methylpiperazin- 1-yl)thiazol-4-yl]ethoxyphenyl)propionic acid, (2S)-3-(4-(benzyloxy)phenyl)-2-((1-methyl-3-oxo-3-phenylpropenyl)amino)propionic acid, (2S)-((2-benzoylphenyl)amino)-3-4-[2-(methylpyridin-2-ylamino)ethoxy]phenylpropionic acid, 3-4-[2-(benzoxazol-2-ylmethylamino)ethoxy]phenyl-(2S)-((2-benzoylphenyl)amino)propanoic acid, or mixtures thereof.

Preferably, before any treatment, including treatment of tumors as described below, it should be tested whether the the mechanism of enhanced expression of ABCG2 by PPARg agonist is valid in the given cells. This can be done easily by the methods described in the examples. First of all it should be checked whether the cells are PPARg positive cells. It they are, addition of an agonist should result in the increased expression of ABCG2 which in turn results in an elevated extrusion of an ABCG2 substrate, e.g. a Hoechst dye or mitoxantrone.

It may occur that the cells are PPARg positive cells, however the increased ABCG2 expression does not occure. One reason for that may lie in the interaction(s) between the various components involved in regulation of hormone mediated processes. For example, in US6387673 it is described that histone deacetylase may associate with hormone receptor complexes and contribute to the repression thereof. Thus, the exposure of a repressed system to histone deacetylase inhibitors relieves this repression.

### Cell therapy

Recently, tumor antigen loaded DCs have been introduced to elicit an anti-cancer immune response as part of cell therapy protocols. DC therapy is often combined with chemotherapy. Our results suggest that the application of a PPARg ligand may protect the antigen presenting cells by selective induction of ABCG2 expression. The findings reported herein will most probably be useful in designing more efficient cell therapy protocols. Obviously, this activation should only be utilized in the case of PPARg negative tumor cells, as PPARg activators may elicit an ABCG2 dependent multi-drug resistance in the tumor cells as well.

Cell therapy methods are well known in the art and the skilled person will be able to apply the findings provided herein in given clinical settings.

In a preferred embodiment, bone marrow transplants are applied. In fact, bone marrow transplants have been safely and successfully performed for many years, primarily for treating leukemia, immune deficiency diseases, severe blood cell diseases, lymphoma and multiple myeloma. Moreover, bone marrow may be obtained through a simple procedure of aspiration, from the patient himself, enabling autologous cell therapy, thus obviating the need for donor matching, circumventing immune rejection and other immunological mismatch risks, as well as avoiding the need for immunosuppressive therapy.

In other embodiment, peripheral blood cells are used as a source of undifferentiated cells to develop appropriate transplants.

A large variety of methods for cell therapy are disclosed e.g. in the following books: "Cell Therapy : Stem Cell Transplantation, Gene Therapy, and Cellular Immunotherapy (Cancer: Clinical Science in Practice)", by George Morstyn (Editor), William P. Sheridan (Editor), Karol Sikora (Series Editor) Cambridge University Press; "Autologous Stem Cell Transplantation: Biological and Clinical Results in Malignancies (Advances in Blood Disorders)", by ANGELO CARELLA, Publisher: Martin Dunitz; 1 edition; "Clinical Bone Marrow and Blood Stem Cell Transplantation" by Kerry Atkinson (Editor), Richard Champlin (Editor), Jerome Ritz (Editor), Willem E. Fibbe (Editor), Per Ljungman (Editor), Malcom K. Brenner (Editor) Publisher: Cambridge University Press; 3 edition; "The BMT Data Book: A Manual for Bone Marrow and Blood Stem Cell Transplantation" by Kerry Atkinson "Cambridge University Press; 1st edition; "Thomas' Hematopoietic Cell Transplantation" by E. Donnall Thomas, Karl G. Blume (Editor), Stephen J. Forman (Editor), Frederick R. Appelbaum (Editor) Publisher: Blackwell Publishing, Incorporated; 3 edition.

### PPARg agonists treatments

In fact, as PPARg agonists, such a rosiglitazone (RSG) are used as drugs for the treatment of various diseases (autoimmune or inflammatory conditions, disturbances of adipogenesis, neoplastic diseases wherein it is used as a differentiating agent), it is contemplated that in patients under chemotherapy with ABCG2 substrate drugs these diseases can be treated more advantagously by PPARg agonists, since the cells affected by the PPARg agonists are at the same time protected against chemotherapy.

It remains to be defined how this mechanism contributes to DC function and activity. It is also not clear what is the function of this pathway under normal, non-pathological conditions. In macrophage ABCA1 and ABCG1 have an important role in the efflux of cholesterol (Repa, J. J. et al (2000) Science 289, 1524-1529; Chawla, A. et al. (2001) Mol Cell 7, 161-171). It is a debated issue whether ABCG2 can transport any lipids. It was reported that ABCG2 mediated the efflux of cholesterol on bacterial cell membrane (Janvilisri, T. et al. (2003) J Biol Chem 278, 20645-20651) but unlike some other members of the ABCG subfamily, ABCG2 seems not to be critically involved in lipid metabolism, because in ABCG2 deficient mice the plasma levels of cholesterol and phospholipids were normal (Jonker, J. W. et al. (2002) Proc Natl Acad Sci U S A 99, 15649-15654). We failed to detect any enhanced cholesterol efflux on PPARg activated monocyte-derived DCs (data not shown). Other multidrug transporters have roles in enhanced DC transmigration and leukotriene production (Randolph, G. J. et al. (1998) Proc Natl Acad Sci U S A 95, 6924-6929; Robbiani, D. F. et al. (2000) Cell 103, 757-768). These data prompted us to investigate the effect of blocked ABCG2 activity in PPARg activated cells. We did not detect major changes in DC phenotype. However, we observed that in the presence of the ABCG2 inhibitor DCs expressed less CD1a. Previous results showed that PPARg activators negatively regulate CD1a (Szatmari, I. et al. (2004) Immunity 21, 95-106; Nencioni, A. et al. (2002) J Immunol 169, 1228-1235), therefore it is formally possible that regulated ABCG2 expression contributes to this by exporting signaling molecules. It is also possible that certain PPARg ligands might be transported by ABCG2 providing a negative feed back regulation to ligand responsiveness.

### EXAMPLES

### Experimental procedures

Ligands-Rosiglitazone, troglitazone and T0901317 were obtained from Alexis Biochemicals, GW9662 and GW347845X were provided by T. M. Willson (GlaxoSmithKline, Research Triangle Park, NC). KO143 was kindly provided by Dr. Gerit-Jan Koomen, Amsterdam.

Plasmids-Mammalian expression vectors coding human retinoid X receptor α (RXRa), mouse PPARg, β-galactosidase and thymidin-kinase (TK)-Luc were described previously (Szanto, A. et al. (2004) Mol Cell Biol 24, 8154-8166). The identified 150 bp fragment of the genomic region of the ABCG2 promoter containing 3 response elements was cloned into TK-Luc to obtain enhancer trap construct.

*Transient transfections and reporter gene assays*-TK-Luc enhancer were transfected along with the indicated receptors. All transfection experiments were performed with COS1 and 293T cells using jetPEI reagent (Qbiogene) according to the manufacturer's instructions. Cells were lysed and assayed for reporter expression 24 h after transfection. The luciferase assay system (Promega) was used according to the manufacturer's instruction. The β-galactosidase activity was determined as described previously (Nagy, L. et al., (1999) Genes Dev 13, 3209-3216). Measurements were made using a Wallac Victor-2, multilabel counter. Luciferase activity of each sample was normalized to the β-galactosidase activity.

*RNA interference*-For siRNA delivery the AMAXA's Nucleofection technology (AMAXA) was applied. MM6 cells (1x10⁶) were harvested and electroporated with 1.5 µg siRNA using the Cell line nucleofector kit V (AMAXA), program v-001. We used a previously described PPARg specific siRNA sequence (Kelly, D. et al. (2004) Nat Immunol 5, 104-112): GCCCTTCACTACTGTTGAC-d(TT) (SEQ ID NO. 10). As a negative control, Silencer Negative Control 1 siRNA (Ambion) was used.

*Chromatine immunoprecipitation*-Chromatine immunoprecipitation was carried out as described previously (Szanto, A. et al. (2004) Mol Cell Biol 24, 8154-8166). Immunoprecipitation was performed with the following monoclonal antibodies: anti-RXRa (2ZK8508H; Perseus Proteomics, Japan), anti-PPARg (2ZK8713; Perseus Proteomics, Japan).

*Electrophoretic mobility shift assays*-Electrophoretic mobility shift assays were performed as described earlier (Szanto, A. et al. (2004) Mol Cell Biol 24, 8154-8166; Benko, S. et al. (2003) J Biol Chem 278, 43797-4380617). In brief, full-length, RXRa, and PPARg receptors were produced by using the T7 Quick TNT in vitro transcription-translation kit (Promega). For supershift experiments, the receptors were preincubated with the indicated monoclonal antibodies: prior to the binding reaction: anti-RXRa (2ZK8508H; Perseus Proteomics, Japan), anti-PPARg (2ZK8713; Perseus Proteomics, Japan). The sequences of oligonucleotides that were used in the competition experiments are as follows:

| name | SEQ ID NO. | sequences (only one strand is shown) |
|---|---|---|
| A element WT | SEQ ID NO. 11 | GGGGTGGAGGGCAGAGGGCAATGGC |
| A element mut | SEQ ID NO. 12 | GGGGTGGGCGAGAGAGGGCAATGGC |
| B element WT | SEQ ID NO. 13 | GGGCACAAGGTTAAAGGTTAAGTGA |
| B element mut | SEQ ID NO. 14 | GGGCACATTGAGAAAGGTTAAGTGA |
| C element WT | SEQ ID NO. 15 | GGTCATTAGGTCAGATGTTAGATGA |
| C element mut | SEQ ID NO. 16 | GGTCATTTCGAGAGATGTTAGATGA |

*Cell culture and ligand treatment*-Monocytes (98% CD14+) were obtained from buffy coats by Ficoll gradient centrifugation and immunomagnetic cell separation using anti-CD14-conjugated microbeads (VarioMACS; Miltenyi Biotec). DCs were prepared as described previously (Szatmari, I. et al. (2004) Immunity 21, 95-106). In brief, monocytes were resuspended into six-well culture dishes at a density of 1.5x10⁶ cells/ml and cultured in RPMI 1640 supplemented with 10% FBS (Invitrogen), containing 800 U/ml GM-CSF (Leucomax) and 500 U/ml IL-4 (Peprotech). Cells were cultured for 5 or 6 days (unless otherwise indicated) and the IL-4 and GM-CSF addition was repeated at day 3. Ligands or vehicle control (50% DMSO/ethanol) were added to the cell culture from the first day on. MonoMac-6 (MM6) cells were a kind gift of E. Duda (Biological Research Center, Szeged, Hungary). These cells were cultured in RPMI 1640 supplemented with 10% FBS.

*Western blot analysis*-50 µg protein whole cell extract was separated by electrophoresis in 10% polyacrylamide gel and then transferred to PVDF membrane (Bio-Rad Laboratories). Membranes were probed with anti-ABCG2 (BXP-21) antibody (Alexis Biochemicals), then stripped and re-probed with anti-GAPDH antibody (Sigma) according to the manufacturer's recommendations.

*FACS analysis*-Cell staining was performed using FITC- or PE-conjugated monoclonal antibodies: Labeled antibodies for flow cytometry included anti-CD1a-PE, CD1d-PE, CD80-PE, CD86-PE and isotype-matched controls (BD PharMingen). For the ABCG2 detection cells were fixed with 1% paraformaldehyde for 5 min, then incubated with unlabeled anti-ABCG2 (5D3) or isotype-matched control (R and D system) antibody for 30 min at 37 °C, followed by staining with FITC-labeled anti-mouse antibody (BD PharMingen). The fluorescence of labeled cells was measured by using a FACSCalibur flow cytometer (Beckton Dickinson).

*Real-time RT-PCR*-Total RNA was isolated with TRIZOL reagent (Invitrogen). Reverse transcription was performed at 42°C for 1 h and 72 °C for 5 min from 100-200 ng of total RNA using Superscript II reverse transcriptase (Invitrogen). Quantitative PCR was performed using real-time PCR (ABI PRISM 7900, Applied Biosystems): 40 cycles at 95°C for 12 s and 60°C for 30 s using Taqman assays. All PCR reactions were done in triplicate with one control reaction containing cDNA that was reverse transcribed without RT enzyme. The comparative Ct method was used to quantify transcripts, and the expression level was normalized to 36B4 or cyclophilin A. The sequences of the primers and probes are available upon request.

*Measurement of Hoechst retention by CLSM and FACS*-DCs were plated on Lab-Tek coverslip chambers. Cells were incubated with 0.5 µg/ml Hoechst 33342 for 1 h at 37 °C with or without KO143 (1 µM), then subjected to microscopic analysis. Hoechst fluorescence was measured on a Zeiss LSM 510 confocal microscope with a Plan-Apochromat 60x (NA 1.4) oil immersion objective using maximal detection pinhole diameter (1 mm) to get signal from the whole depth of the nucleus. Hoechst was excited by the 351 and 364 nm lines of an Ar ion laser, emission was measured through a 385-to-470 nm bandpass filter.

Hoechst content was also measured on a Becton Dickinson FACSVantage flow cytometer. Hoechst fluorescence was excited by the 351 and 364 nm lines and the blue and red Hoechst signals were detected through 402-to-446 nm and >650 nm filters, respectively.

*Measurement of mitoxantrone uptake by FACS analyses*-Cells were resuspended in phenol red free HPMI medium containing the desired fluorescent compound (20 µM mitoxantrone) with or without KO143 (1 µM) and incubated at 37 °C for 30 min, Thereafter the cells were washed with cold HPMI and subjected to flow cytometric analyses. The cells were assessed for fluorescence intensity with a FACSCalibur cytometer (Beckton Dickinson).

*Cytotoxicity assay*-Cytotoxicity assays were carried out using the MTT assay. Briefly, cells were plated in 96-well plates at a density of 10⁵ cells per well. Mitoxantrone at various concentrations with or without KO143 was added to DCs and cells were incubated at 37 °C for 48 h. After incubation, cytotoxicity was assessed by using the MTT assay. The MTT [3-(4,5-dimethylthiazolyl-2)-2,5-diphenyl-tetrazolium bromide] assay was performed as described previously (Gerlier, D. et al. (1986) J Immunol Methods 94, 57-63) and as specified by the manual of American Type Culture Collection (ATCC).

*Statistical analyses*-Values are expressed mean ± SD of the mean. Significant differences between mean values were evaluated using two-tailed, unpaired Student's t test.

### ABCG2 is highly and selectively induced by PPARg agonists in human dendritic cells

Previously we have characterized the role of PPARg nuclear receptor in monocyte-derived dendritic cell (DC) differentiation (Szatmari, I. et al. (2004) Immunity 21, 95-106). We observed that PPARg was acutely and rapidly upregulated during DC differentiation and acted as a modifier of DC gene expression and function. This cellular system is particularly suitable to identify PPARg responsive genes because the bona fide PPARg target, the fatty acid binding protein 4 (FABP4 also known as aP2) was highly induced (50-100 fold) upon PPARg agonist treatment. We sought to identify direct targets of PPARg with expression pattern similar to that of FABP4 using global gene expression analysis (Affymetrix microarrays). One of the candidate genes matching these criteria was an ABC half transporter, ABCG2. In order to validate the microarray data the transcript level of ABCG2 was determined by real-time quantitative RT-PCR (RT-Q-PCR) from RNAs obtained from monocytes and monocyte-derived human DCs. When compared the expression of FABP4 to ABCG2 a similar pattern of mRNA levels was observed (Fig. 1*A*). Consistent with the microarray data, PPARg agonist (rosiglitazone, abbreviated as RSG) treatment highly enhanced the expression of both genes. Although rosiglitazone is a specific PPARg agonist, its ability to induce ABCG2 in developing DCs still may be related to other pharmacologic activities. In order to address this issue a PPARg antagonist (GW9662) was simultaneously added to the cells. As shown in Fig. 1A, administration of the antagonist (ANT) abolished the induction of ABCG2 suggesting that this regulation is indeed PPARg receptor dependent. We used other chemically distinct synthetic PPARg agonists to further establish the receptor specificity of ABCG2 induction. Cells were treated with troglitazone (TRO) or with GW347845X (abbreviated as GW7845). As shown in Fig. 1*B* all of these PPARg activators induced FABP4 and ABCG2 expression.

Similar results were obtained in normal macrophages (Fig. 9) by microarray (Affymetrix) measurements and by quantitative RT-PCR. Differenciating cells, if a PPARg agonist, rosiglitazone was added, showed a significan increase in ABDG2 expression already after 6 hours of treatment.

These data suggest that this mechanism probably exists in a variety of immune cells.

### Upregulation of ABCG2 in a leukemia cell line

Upregulation of ABCG2 was also detected in a monocytic/macrophage leukemia cell line (Monomac 6, abbreviated as MM6) by the activation of the PPARg:RXR heterodimer (Fig. 1C). PPARg forms a heterodimer with the RXR receptor. Interestingly, in MM6 cells both components of the heterodimer had to be activated to elicit a profound ABCG2 expression induction (MM6 cells were simultaneous treated with 2.5 µM RSG and 100 nM LG268, an RXR agonist). To further establish the role of PPARg in ABCG2 regulation, we used RNA interference to suppress the expression of PPARg in MM6 cells. We used a previously described (Kelly, D. et al. (2004) Nat Immunol 5, 104-112) PPARg-specific siRNA that effectively inhibited the expression of endogenous PPARg but it did not interfere with expression of PPARd (Fig. 1*C*). Significantly, PPARg specific siRNA treated cells showed impaired induction of FABP4 and ABCG2, indicating that the transcriptional induction of these genes are receptor dependent. Taken together, these results demonstrate that activation of PPARg induces elevated ABCG2 mRNA expression both in DCs and in the myeloid leukemia cell line MM6. Moreover, this regulation appears to be selectively PPARg receptor dependent in both cell types.

Thus, in a neoplastic cell which is multi-drug resistant due to overexpression of ABCG2 according to the mechanism shown herein, silencing PPARg can result in a decrease or cease of multidrug resistance.

### ABCG2 is regulated by PPARg in a mechanism independent of LXR

It has been documented that, under certain conditions, PPARg can indirectly activate gene expression by the induction of its direct target receptor, LXRa. As an example, ABCA1 expression is regulated via such a mechanism (Chawla, A. et al. (2001) Nat Med 7, 48-52; Chawla, A. et al. (2001) Mol Cell 7, 161-171). A previous study has also established that ABCA1 and ABCG1 are direct target genes of LXRs (Repa, J. J. et al. (2000) Science 289, 1524-1529). We also observed that a PPARg ligand enhances the expression of LXRa in DCs (data not shown) suggesting that an LXR dependent pathway may be responsible for the up-regulation of certain ABC transporters.

In order to examine if the activation of PPARg enhances ABCG2 transcription via the induction of LXRa, we compared the effects of PPARg and LXR agonists on the mRNA levels of ABCA1, ABCG and ABCG2 by RT-Q-PCR. As shown in Fig. 2*A*, we found that in this cell type the activation of PPARg failed to induce ABCA1 and ABCG1 expression, while we detected a profound upregulation of these transporter mRNAs if cells were treated with an LXR specific agonist (T0901317). In contrast to ABCA1 and ABCG1, T0901317 did not affect the expression of ABCG2. These data indicate that in human DCs ABCG2 regulation is independent from LXRa induction/activation, and in these cells the selective activation of nuclear hormone receptors results in a non-overlapping ABCA1- ABCG1 vs ABCG2 expression pattern.

### PPARg directly regulates the transciption of the ABCG2 gene

Our results demonstrated that the mRNA expression of ABCG2 is positively regulated by PPARg specific ligands in a receptor dependent manner. Next, we have examined the dose and time dependence of ABCG2 induction. PPARg activators (for example rosiglitazone) belong to a class of antidiabetic compounds (thiazolidinediones=TZD), that are used in the clinic to treat type II diabetes (Willson, T. M. et al. (2001) Annu Rev Biochem 70, 341-367). The plasma concentration of rosiglitazone (RSG) in patients treated with 4 mg RSG is between 50-350 ng/ml (100-1000 nM) (Niemi, M. et al. (2004) Clin Pharmacol Ther 76, 239-249). To examine whether ABCG2 can be activated ex vivo in this concentration range, we performed dose response experiments. As shown in Fig. 2B 100 nM RSG readily induced the expression of ABCG2, suggesting that this gene is to be induced in DCs in vivo upon RSG treatment.

Next we asked whether the activation of PPARg directly elicits the accumulation of ABCG2 transcripts. Time-course experiments (Fig. 2C) revealed that ABCG2 similarly to FABP4 were rapidly upregulated upon PPARg ligand administration. This finding strongly suggests that PPARg directly regulates the expression of ABCG2.

### The promoter sequence of the ABCG2 gene comprises putative PPAR responsive elements

To test further this possibility, we analyzed the promoter sequence of the ABCG2 gene to find putative PPAR responsive elements. The sequence analysis of the previously reported promoter region (-1285/+362) of the human ABCG2 gene (Krishnamurthy, P. et al. (2004) J Biol Chem 279, 24218-24225) did not reveal any canonical PPAR response elements. Furthermore transient transfection assays carried out to assess the activity of this promoter region (-1285/+362) failed to indicate any PPARg dependent transcriptional response (data not shown). Therefore we decided to use a bioinformatics approach for the identification of conserved promoter regions and putative PPAREs. The ABCG2 protein is relatively well conserved in evolutionary distant animals. However the completely untranslated first exon shows very little similarity between species, thus the identification of the promoter region is rather difficult. In addition several species such as the mouse and rat contain paralogous ABCG2 genes with alternatively spliced 5' UTR exon(s). Using cDNA sequences to identify the first UTR exons we have succeeded in finding putative promoter regions of bovine and dog ABCG2 genes beside the almost identical primate sequences. Comparison of 5000 basepairs of human, dog and bovine ABCG2 promoter regions (counting from the position of the beginning of the 5' longest cDNA sequence) revealed a well conserved region (SFig. 3D and table 1). We have identified a 150 bp portion (-3946/-3796) of this conserved region, which contains three potential PPAR response elements in the human and one in bovine and dog (table1, Fig. 3A and SFig. 3*D*).

**Table 1**

| | | |
|---|---|---|
| Hs150bp | (SEQ ID NO 6) | |
| Cf150bp | (SEQ ID NO 7) | |
| Bt150bp | (SEQ ID NO 8) | |

To test the ability of these elements for binding PPARg:RXR in vitro, we carried out electrophoretic mobility shift assays (EMSA). The EMSA analysis revealed that all three putative elements, containing direct repeat 1 (DR1=AGGTCANAGGTCA; SEQ ID NO 9) motifs, were able to specifically bind PPARg:RXR heterodimers (Fig. 3*B*). Element A is positioned in the normal 5'-3' direction while elements B and C are positioned in the reverse direction. In these experiments we also found that if the two receptors were added together, a stronger specific binding was observed (lane PPARg/RXRa on Fig 3*B*). This binding was effectively competed by either unlabeled (cold) oligos, or a canonical DR1 oligonucleotide (Cold WT and Cold DR1, respectively). Consequently, the band indicating binding of PPARg/RXRa heterodimer is faded. No such competition was observed with oligonucleotides containing mutations (Cold MUT). The specificity of the binding was further demonstrated by the use of antibodies against RXRα and PPARg, which induced marked supershifts (lanes Anti-PPARg and Anti-RXRa in Fig. 3*B*), i.e. the motility of these bands is further reduced. With elements B and C aspecific binding could be detected; however this was present in the blank samples as well.

Next an enhancer trap method was employed to assess the binding and transcription activation capacity of these elements. We introduced the identified element (-3946/-3796) into a reporter plasmid upstream of the minimal TK-luciferase promoter, generating the heterologous promoter construct termed ABCG2enhancer-TK-Luc (TK-Luc ENH). To determine the receptor binding capacity of the enhancer region in a cellular system, we co-transfected the ABCG2enhancer-TK-Luc reporter construct with the expression vector, containing constitutively active PPARg (VP16-PPARg) alone or VP16-PPARg along with RXRα. We obtained a robust induction of reporter gene activity when the cells were cotransfected with VP16-PPARg and, as anticipated, the induction was further increased in the presence of LXRA (Fig. 4*A*). We obtained similar results in two independent cell lines (COS1 and 293T). In a specificity control experiment, the TK-Luc dependent transcription was not modified by receptor expression in the absence of the enhancer region. These data provided evidence for a possible *in vivo* binding of the receptor heterodimer to the identified enhancer elements in the ABCG2 gene.

To test this role directly, COS1 cells were co-trasfected with the ABCG2enhacer-TK-Luc reporter construct, along with expression vectors coding for PPARg or RXRα in various combinations (Fig. 4*B*). We observed that the presence of the PPARg:RXR heterodimer induced a major transcription response, and this was further elevated by the administration of a PPARg specific ligand, RSG. We also included a promoter element containing canonical PPAR responsive elements (3xPPRE) as a positive control (Fig. 4*B*). Finally, we analyzed if PPARg and RXRα protein was bound to the ABCG2 enhancer region in cells using chromatin immunoprecipitation (ChIP) combined with real-time PCR. Chromatin was obtained from PPARg transduced 293T cells. A genomic region of the LXRa gene containing a PPRE element was used as a positive control and a genomic region of the 36B4 gene was tested as a negative control. Consistent with EMSA and the transient tranfection results our analyses demonstrated that both PPARg and RXRα were present on the ABCG2 enhancer region in intact cells (Fig. 4*C*).

Taken together, all these data indicate that the genomic region examined, upstream of the ABCG2 coding sequence, can bind PPARg:RXR heterodimers, and this enhancer region is able to confer a PPARg dependent transcriptional response. Thus this genomic region most likely participates in the PPARg dependent regulation of ABCG2 expression. Moreover, this mechanism works in a cell-type independent manner.

The above results also show that by specific blocking of the newly found PPARg responsive elements the binding of the PPARg:RXR heterodimers and thereby the enhancing mechanism described above can be obviated.

### ABCG2 protein expression is upregulated in PPARg activated cells

Our results described above show that PPARg directly and specifically induces the mRNA expression of ABCG2 in monocyte-derived DCs. In order to estimate the changes in the protein level and the cell surface expression of ABCG2 in DCs, we performed antibody staining in Western blot and in flow-cytometry experiments. By using the BXP 21, ABCG2 specific monoclonal antibody in Western blotting, we could not detect the ABCG2 protein either in human monocytes or untreated DCs. However, a specific 70 kDa protein product appeared on the immunoblot when human DCs were pre-treated with a PPARg ligand, RSG (Fig. 5*A*). We have also determined the cell surface expression of the ABCG2 protein by flow-cytometry (Fig. 5*B*), using the 5D3 monoclonal antibody, which reacts with an extracellular epitope of the transporter. Consistent with the mRNA expression and Western blot data, significant cell surface expression of the ABCG2 protein was detected only in the RSG treated DCs, while this expression was reduced if the cells were treated with a PPARg antagonist (GW9662). All these data clearly indicate that PPARg-dependent activation of transcription results in an enhanced ABCG2 protein production, which is correctly processed to reach the cell membrane surface in DCs. Next we investigated the functional consequences of the elevated level of this xenobiotic transporter on DCs.

### PPARg instructed DCs have an enhanced capacity to extrude xenobiotics and acquire resistance against anticancer agent

The ABCG2 transporter has a wide substrate recognition and transport capacity and, by performing an efficient extrusion of the Hoechst 33342 fluorescent dye, the expression of ABCG2 has been implicated in the so called side population phenotype of stem cells (Zhou, S. et al. (2001) Nat Med 7, 1028-1034, Scharenberg, C. W. et al. (2002) Blood 99, 507-512). With the aim of correlating ABCG2 expression level and the function of this transporter, Hoechst 33342 dye accumulation was compared in normal and PPARg agonist treated DCs, respectively. We have monitored the nuclear Hoechst dye fluorescence with confocal microscopy and flow cytometry analyses. As shown in Fig. 6, *A* and *B*, most of the PPARg activated DCs accumulated less Hoechst dye than the non-stimulated control cells, indicating an enhanced pump activity of ABCG2. In order to assess the specificity of the assay, KO143 a specific and selective inhibitor of ABCG2, was added to block the transporter activity. We found that the reduced accumulation of the fluorescent dye in the stimulated DCs was ABCG2 dependent, because the administration of KO143 abolished the effect of the PPARg ligand treatment. Next we assessed the mitoxantrone efflux capacity of the PPARg activated DCs. As shown in Fig. 7*A*, mitoxantrone (MX) accumulation was rapid in the control DCs, while after the addition of the PPARg activator RSG, the cells accumulated less mitoxantrone than the control DCs. Similarly to that found in the Hoechst dye uptake studies, mitoxantrone extrusion by ABCG2 in the activated cells was fully inhibited by the specific inhibitor of the pump, KO143 Next we examined whether the presence of ABCG2 in RSG treated DCs conferred a protective effect against the cytotoxic drug, mitoxantrone. DCs were treated with different doses of mitoxantrone for 48 h and then cell viability was assessed by MTT assay. As shown in Fig. 7B, while low doses of MX induced cell death in the control DCs, the PPARg ligand treated DCs were significantly less sensitive to this cytotoxic agent. The addition of ABCG2 inhibitor KO143 removed this protective effect. In agreement with the enhanced expression of ABCG2 after PPARg activation of DCs, the observed reduced Hoechst dye and drug accumulation, as well as the reduced sensitivity to MX in these cells indicate that ABCG2 is fully functional, and activated DCs acquire a protection against cytotoxic agents.

### Blocking of ABCG2 only mildly modified the DC phenotype

In accordance with the results provided herein PPARg activated DCs have an enhanced transport capacity to extrude various chemically synthesized drugs. It is an intriguing question, what is the physiological role of this transporter on DCs. To further assess the functional consequence of the elevated expression of ABCG2, including its effect on gene expression, we cultured the PPARg activated cells in the presence of ABCG2 inhibitor (KO143) and compared the phenotype of the inhibitor treated and control cells. We and others have recently characterized the phenotype of the PPARg instructed DCs. Consistent with the previous findings (Szatmari, I. et al. (2004) Immunity 21, 95-106; Gosset, P. et al. (2001) Eur J Immunol 31, 2857-2865; Nencioni, A. et al. (2002) J Immunol 169, 1228-1235), PPARg activated DCs express more CD86 and CD1d, but the expression of CD80 and CD1a are decreased (Fig. 8*A*). The administration of the selective ABCG2 inhibitor (KO143) did not change the cell surface expression of CD80, CD86 or CD1d but the expression of CD1a was slightly decreased on the PPARg activated cells (Fig. 8*A*). We also assessed the mRNA expression of CD1a and FABP4 by RT- Q-PCR. The transcript level of FABP4 did not change but the CD1a expression was reduced consistent with the changes detected on the cell surface expression. Together our data indicate that blocking the ABCG2 transporter in the PPARg instructed DCs only mildly modify the phenotype of DCs and it is unlikely that it contributes significantly to PPARg-dependent changes in DC phenotype. However, a subset of PPARg regulated genes was slightly modified when the pump was inhibited. This raises the issues that ABCG2 might have some regulatory roles in DCs besides its established cytoprotective function, which is likely to be part of an adaptive response.

### Autologous cell transplantation of a patient in need of increasing immunological tolerance, e.g. n a post-transplant condition

*Ex vivo* manipulation of DCs allows the DCs to re-induce or maintain immunological tolerance, or to stimulate cellular immune responses or modulate inflammatory or anti-inflammatory mechanisms, as disclosed e.g. in W02004/101776 (Nagy L. et al.). In such a treatment the mechanism disclosed herein can be utilized to select the cells of modified phenotype, for example as follows.
1) Autologous DC precursors (monocytes) are separated from the peripheral blood of patients by magnetic or other isolation techniques.
2) DC precursors are differentiated *ex vivo* in the presence of a cytokine cocktail comprising GM-CSF and IL-4 and optionally other cytokines.
3) *Ex vivo* DC maturation is targeted by RSG to generate the desired phenotype and function. In this case the level of CD1d (a CD1 type I molecule) will be increased whereas CD1a (a CD1 type I molecule) will be down regulated within a few days. Optionally, an RXR agonist is also added to the cells which further increases the effect achieved by RSG. The same drugs, as disclosed above, also induce the expression of ABCG2. Depending of the treatment regime, ABCG2 expression occures within 24 hours after treatment with RSG, whereas the alteration in the expression of the CD1 molecules can be observed in a few days. Addition of an RXR agonist will probably accelerate this process.
4) Dendritic cells of altered phenotype are selected by a cytotoxic drug transportable by ABCG2, e.g. mitoxantrone. The selective drug may be added together with RSG or e.g. in 6 to 48 hours, i.e. soon after the enhancement of the ABCG2 expression.
5) Mature dendritic cells having the desired expression profile (CD1d up-regulated and CD1a downregulated) are re-introduced into the patient, wherein immunological tolerance is effected by recruiting CD1 type II restricted iNKT cells and/or increasing their activities.

### Autologous cell transplantation of a patient with a hematological tumor

In this example a patient with hematological tumour is treated by a manipulated dendritic cells, wherein said patient is receiving chemotherapy comprising administration of a drug transportable by ABCG2.

Specific methods wherein chemotherapy of tumors and autologous cell therapy are combined are disclosed e.g. in WO9924045A1 and documents cited therein. Treatment of patients suffering in hematological tumors by a therapy comprising administration of manipulated dendritic cells is taught in WO2004/101776 (Nagy L. et al.). In this example a personalized therapy is carried out. First it is to be tested in vitro whether iNKT cells have anti-tumor NK-cell-like activity in the given disease (Wilson S. B. and Delowitch T. L., Nature Reviews Immunology, 3, (2003) 211).
- Peripheral blood mononuclear cells (PBMCs) are collected by leukapheresis from a patient receiving dose-intensive therapy, then CD34+ progenitor cells are selected.
- CD34+ cells are differentiated *ex vivo* in the presence of a cytokine cocktail comprising GM-CSF and IL-4 and optionally other cytokines.
- *Ex vivo* DC maturation is targeted by RSG to generate cells of an increased level of CD1d (a CD1 type I molecule) will be increased whereas CD1a (a CD1 type I molecule) will be down regulated within a few days. RSG also induces the expression of ABCG2 which confers cytoprotection for the treated cells.
- Mature dendritic cells having the desired expression profile are re-introduced into the patient, wherein addition of dendritic cells result in an activation of iNKT cells providing an anti-tumor effect. If needed, alpha-GalCer is administered additionally.

In personalized DC-based tumor-specific vaccination, besides the steps described above, preferably tumor associated antigens may be selected and isolated in a sufficient amounts either from self tumor tissue or as a recombinant protein or synthetic peptide for loading immature DC. Thereafter loading with tumor antigens and the appropriate activation of autologous DC is carried out.

An abundant description of various practical methods applicable for isolation, cultivation, differentiation and reintroduction of blood cells in case of autologous cell transplantation including treatment of neoplastic diseases can be found in the art, e.g. in "The BMT Data Book: A Manual for Bone Marrow and Blood Stem Cell Transplantation", by Kerry Atkinson ", Cambridge University Press; 1st edition; and in "Thomas' Hematopoietic Cell Transplantation, by E. Donnall Thomas, Karl G. Blume (Editor), Stephen J. Forman (Editor), Frederick R. Appelbaum (Editor), Blackwell Publishing, Incorporated; 3 edition. An exemplary method is carried out as follows.

In summary, our study revealed that PPARg activated DCs and macrophages express an increased level of functional ABCG2 transporter and these cells acquire relative protection against endo- and xenobiotics. These features have important consequences to the survival and drug resistance of human dendritic cells during immune regulation.

We have also identified and characterized regions of the ABCG2 gene involved in this transcriptional activation.

These findings show that combined treatments by PPARg agonists and cytotoxic drugs provide unexpected advantages, and allow a series of various treatment type in the field of neoplastic diseases as well as cell therapy, including autologous cell therapy. Moreover our results have a significant impact on research both in the field of PPARg and RXR agonists as well as ABCG transporters.

## Claims

1. A combination of a PPARg (peroxisome proliferator activated receptor gamma) agonist and a chemotherapeutic drug transportable by ABCG2 (ATP binding cassette transporter G2), for use in protecting somatic mammalian cells from the chemotherapeutic drug in a therapy.

2. A pharmaceutical kit for use in therapy as defined in claim 1, said kit comprising at least a PPARg agonist or composition thereof, and a chemotherapeutic drug transportable by ABCG2 or a composition thereof.

3. The combination or kit for use according to any of claims 1 or 2, wherein said cells are immune cells.

4. The combination or kit for use according to any of claims 1 to 3, wherein the disease is at least one of the following diseases: a neoplastic disease or an immunological disease, and
the therapy comprises cell therapy and/or chemotherapy.

5. The combination or kit for use according to claim 4, wherein the disease is a neoplastic disease and wherein the neoplastic cells are PPARg negative cells and/or the neoplastic cells do not overexpress ABCG2.

6. Use of a PPARg agonist in the manufacture of a medicament for use in the treatment of a neoplastic disease, wherein the treatment of the neoplastic disease comprises both cell therapy and chemotherapy by a chemotherapeutic drug transportable by ABCG2 and the neoplastic cells of the patient are PPARg negative cells.

7. The combination or kit according to any of claims 1 to 5 for use in a method for combined treatment of a neoplastic disease of a patient by cell therapy and chemotherapy, wherein the neoplastic cells of the patient are PPARg negative cells, said method comprising the steps of
- isolating cells wherein expression of ABCG2 is inducible by PPARg or PPARg:RXR heterodimer and preferably the cells are PPARg positive cells, from a subject, wherein it is optionally tested whether expression of ABCG2 is inducible in said cells by PPARg or by PPARg:RXR heterodimer,
- activating PPARg or increasing the expression of PPARg by the PPARg agonist and, if desired, RXR in said cells, thereby inducing or enhancing the expression of ABCG2 in said cells,
- optionally selecting the cells by a cytotoxic drug transportable by ABCG2,
- differentiating the cells to immature or mature cells and
- introducing the differentiated cells into a patient
- treating the patient by a chemotherapeutic drug transportable by ABCG2,
wherein preferably the subject the cells are isolated from is identical with the treated patient.

8. The combination or kit according to any of claims 1 to 5 for use in a method for cell therapy wherein therapeutic cells are selected before administration, said method comprising the steps of
- isolating cells wherein expression of ABCG2 is inducible by PPARg or PPARg:RXR heterodimer and preferably the cells are PPARg positive cells, from a subject, wherein it is optionally tested whether expression of ABCG2 is inducible in said cells by PPARg or PPARg:RXR heterodimer,
- activating PPARg or increasing the expression of PPARg by the PPARg agonist and, if desired, RXR in said cells, thereby effecting a desired alteration in the phenotype of the cells and inducing or enhancing the expression of ABCG2 in the same cells,
- selecting the cells expressing ABCG2 and having the altered phenotype by a cytotoxic drug transportable by ABCG2,
- differentiating the cells to immature or mature cells and
- introducing the differentiated cells into a patient,
wherein preferably the subject the cells are isolated from is identical with the treated patient.

9. The combination or kit for use as in claims 7 or 8 wherein the isolated cells to be treated are antigen presenting cells (APCs) or precursor cells of an APC, preferably monocytes, more preferably dendritic cells (DCs) and
PPARg and, if desired, RXR is activated in said cells by administering to the cells a PPARg agonist and, if desired, an RXR agonist.

10. An in-vitro process for protecting somatic mammalian cells against the effect of a cytotoxic drug transportable by ABCG2, comprising the steps of
- providing said cells in an isolated form,
- enhancing the expression of ABCG2 by activating or increasing the expression of PPARg and, if desired, RXR in said cells,
- exposing the cells to the effect of a cytotoxic drug transportable by ABCG2.

11. The process according to claim 10 wherein PPARg is activated by administering to the cells a PPARg agonist, and optionally RXR is activated by administering to the cells an RXR agonist.

12. The process according to claim 10, wherein expression of PPARg and, if desired, of RXR is effected by gene transfer into the therapeutic cells.

13. A PPARg agonist for use in conferring cytoprotection for mammalian immune cells against a cytotoxic drug transportable by ABCG2.

## Patentansprüche

1. Eine Kombination aus einem PPARg- (Peroxisom-Proliferator-aktivierter Rezeptor Gamma) - Agonisten und einem Chemotherapeutikum, das durch ABCG2 (A'TP-bindender Kassettentransporter G2) transportierbar ist, zur Verwendung beim Schützen von somatischen Säugetierzellen vor dem Chemotherapeutikum in einer Therapie.

2. Ein pharmazeutisches Kit zur Verwendung in einer Therapie wie in Anspruch 1 definiert, wobei das Kit mindestens einen PPARg-Agonisten oder eine Zusammensetzung davon und ein durch AGCG2 transportierbares Chemotherapeutikum oder eine Zusammensetzung davon umfasst.

3. Die Kombination oder das Kit zur Verwendung gemäß einem der Ansprüche 1 oder 2, wobei die Zellen Immunzellen sind.

4. Die Kombination oder das Kit zur Verwendung gemäß einem der Ansprüche 1 bis 3, wobei die Erkrankung mindestens eine der folgenden Erkrankungen ist: eine neoplastische Erkrankung oder eine immunologische Erkrankung, und die Therapie Zelltherapie und / oder Chemotherapie umfasst.

5. Die Kombination oder das Kit zur Verwendung gemäß Anspruch 4, wobei die Erkrankung eine neoplastische Erkrankung ist und wobei die neoplastischen Zellen PPARg-negative Zellen sind und / oder die neoplastischen Zellen ABCG2 nicht überexprimieren.

6. Verwendung eines PPARg-Agonisten bei der Herstellung eines Medikaments zur Verwendung bei der Behandlung einer neoplastischen Erkrankung, wobei die Behandlung der neoplastischen Erkrankung sowohl Zelltherapie als auch Chemotherapie durch ein durch ABCG2 transportierbares Chemotherapeutikum umfasst und die neoplastischen Zellen des Patienten PPARg-negative Zellen sind.

7. Die Kombination oder das Kit gemäß irgendeinem der Ansprüche 1 bis 5 zur Verwendung bei einem Verfahren zur kombinierten Behandlung einer neoplastischen Erkrankung eines Patienten durch Zelltherapie und Chemotherapie, wobei die neoplastischen Zellen des Patienten PPARg-negative Zellen sind, wobei das Verfahren die folgenden Schritte umfasst:
- Isolieren von Zellen aus einer Person, wobei die Expression von ABCG2 durch PPARg oder PPARg:RXR-Heterodimer induzierbar ist und die Zellen vorzugsweise PPARg-positive Zellen sind, wobei optional getestet wird, ob die Expression von ABCG2 in den Zellen durch PPARg oder durch PPARg:RXR-Heterodimer induzierbar ist,
- Aktivieren von PPARg oder Erhöhen der Expression von PPARg durch den PPARg-Agonisten, und, falls gewünscht, von RXR in den Zellen, wodurch die Expression von ABCG2 in den Zellen induziert oder verbessert wird,
- wahlweise Selektieren der Zellen durch ein durch ABCG2 transportierbares zytotoxisches Medikament,
- Differenzieren der Zellen in unreife oder reife Zellen, und
- Einbringen der differenzierten Zellen in einen Patienten,
- Behandeln des Patienten mit einem durch ABCG2 transportierbaren Chemotherapeutikum,
wobei die Person, aus der die Zellen isoliert werden, vorzugsweise mit dem behandelten Patienten identisch ist.

8. Die Kombination oder das Kit gemäß einem der Ansprüche 1 bis 5 zur Verwendung in einem Verfahren zur Zelltherapie, wobei therapeutische Zellen vor der Verabreichung ausgewählt werden, wobei das Verfahren die folgenden Schritte umfasst:
- Isolieren von Zellen aus einer Person, wobei die Expression von ABCG2 durch PPARG, oder PPARg:RXR-Heterodimer induzierbar ist und die Zellen vorzugsweise PPARg-positive Zellen sind, wobei optional getestet wird, ob die Expression von ABCG2 in den Zellen durch PPARg oder PPARg:RXR-Heterodimer induzierbar ist,
- Aktivieren von PPARg oder Erhöhen der Expression von PPARg durch den PPARg-Agonisten, und, falls gewünscht, von RXR in den Zellen, wodurch eine gewünschte Änderung des Phänotyps der Zellen bewirkt wird und die Expression von ABCG2 in denselben Zellen induziert oder verbessert wird,
- Selektieren der Zellen, die ABCG2 exprimieren und den veränderten Phänotyp aufweisen, durch ein durch ABCG2 transportierbares zytotoxisches Medikament,
- Differenzieren der Zellen in unreife oder reife Zellen, und
- Einbringen der differenzierten Zellen in einen Patienten,
wobei die Person, aus der die Zellen isoliert werden, vorzugsweise identisch mit dem behandelten Patienten ist.

9. Die Kombination oder das Kit zur Verwendung wie in den Ansprüchen 7 oder 8, wobei die zu behandelnden isolierten Zellen antigenpräsentierende Zellen (APCs) oder Vorläuferzellen einer APC, vorzugsweise Monozyten, auf noch bevorzugtere Weise dendritische Zellen (DCs) sind, und PPARg und, falls gewünscht, RXR in den Zellen aktiviert wird durch Verabreichen eines PPARg-Agonisten und, falls gewünscht, eines RXR-Agonisten an die Zellen.

10. Ein In-vitro-Verfahren zum Schützen von somatischen Säugetierzellen vor der Wirkung eines durch ABCG2 transportierbaren zytotoxischen Medikaments, umfassend die folgenden Schritte:
- Bereitstellen der Zellen in einer isolierten Form,
- Verbessern der Expression von ABCG2 durch Aktivieren oder Erhöhen der Expression von PPARg und, falls gewünscht, von RXR in den Zellen,
- Aussetzen der Zellen der Wirkung eines durch ABCG2 transportierbaren zytotoxischen Medikaments.

11. Das Verfahren gemäß Anspruch 10, wobei PPARg durch Verabreichen eines PPARg-Agonisten an die Zellen aktiviert wird und optional RXR durch Verabreichen eines RXR-Agonisten an die Zellen aktiviert wird.

12. Das Verfahren gemäß Anspruch 10, wobei die Expression von PPARg und, falls gewünscht, von RXR durch Centransfer in die therapeutischen Zellen erfolgt.

13. Ein PPARg-Agonist zur Verwendung beim Verleihen von Zellschutz an Säugetier-Immunzellen gegenüber einem durch ABCG2 transportierbaren zytotoxischen Medikament.

## Revendications

1. Combinaison d'un agoniste du PPARg (récepteur d'activation et de prolifération des peroxysomes gamma) et d'un médicament chimiothérapeutique pouvant être transporté par l'ABCG2 (transporteur de la cassette de liaison de l'ATP G2), pour une utilisation dans la protection des cellules mammaliennes somatiques contre le médicament chimiothérapeutique dans une thérapie.

2. Kit pharmaceutique pour une utilisation en thérapie selon la revendication 1, ledit kit comprenant au moins un agoniste du PPARg ou une composition de celui-ci et un médicament chimiothérapeutique pouvant être transporté par l'ABCG2 ou une composition de celui-ci.

3. Combinaison ou kit pour une utilisation selon l'une quelconque des revendications 1 ou 2, où lesdites cellules sont des cellules immunitaires.

4. Combinaison ou kit pour une utilisation selon l'une quelconque des revendications 1 à 3, où la maladie est au moins l'une des maladies suivantes : une maladie néoplasique ou une maladie immunologique, et
la thérapie comprend une thérapie cellulaire et/ou une chimiothérapie.

5. Combinaison ou kit pour une utilisation selon la revendication 4, où la maladie est une maladie néoplasique et où les cellules néoplasiques sont des cellules négatives pour le PPARg et/ou les cellules néoplasiques ne surexpriment pas ABCG2.

6. Utilisation d'un agoniste du PPARg dans la fabrication d'un médicament pour une utilisation dans le traitement d'une maladie néoplasique, où le traitement de la maladie néoplasique comprend à la fois une thérapie cellulaire et une chimiothérapie par un médicament chimiothérapeutique pouvant être transporté par l'ABCG2 et les cellules néoplasiques du patient sont des cellules négatives pour le PPARg.

7. Combinaison ou kit selon l'une quelconque des revendications 1 à 5, pour une utilisation dans un procédé pour un traitement combiné d'une maladie néoplasique d'un patient par une thérapie cellulaire et une chimiothérapie, où les cellules néoplasiques du patient sont des cellules négatives pour le PPARg, ledit procédé comprenant les étapes consistant à
- isoler les cellules dans lesquelles l'expression de l'ABCG2 est inductible par le PPARg ou l'hétérodimère PPARg/RXR et de préférence les cellules sont des cellules positives pour le PPARg, à partir d'un sujet, où il est éventuellement testé si l'expression de l'ABCG2 est inductible dans lesdites cellules par le PPARg ou par l'hétérodimère PPARg/RXR,
- activer le PPARg ou augmenter l'expression du PPARg par l'agoniste du PPARg et, le cas échéant, le RXR dans lesdites cellules, induisant ou amplifiant de cette manière l'expression de l'ABCG2 dans lesdites cellules,
- sélectionner éventuellement les cellules par un médicament cytotoxique pouvant être transporté par l'ABCG2,
- différencier les cellules en cellules immatures ou matures et
- introduire les cellules différenciées chez un patient
- traiter le patient par un médicament chimiothérapeutique pouvant être transporté par l'ABCG2,
où de préférence le sujet à partir duquel les cellules sont isolées est identique au patient traité.

8. Combinaison ou kit selon l'une quelconque des revendications 1 à 5, pour une utilisation dans un procédé pour une thérapie cellulaire dans laquelle les cellules thérapeutiques sont sélectionnées avant l'administration, ledit procédé comprenant les étapes consistant à
- isoler les cellules dans lesquelles l'expression de l'ABCG2 est inductible par le PPARg ou l'hétérodimère PPARg/RXR et de préférence les cellules sont des cellules positives pour le PPARg, à partir d'un sujet, où il est éventuellement testé si l'expression de l'ABCG2 est inductible dans lesdites cellules par le PPARg ou l'hétérodimère PPARg/RXR,
- activer le PPARg ou augmenter l'expression du PPARg par l'agoniste du PPARg et, le cas échéant, le RXR dans lesdites cellules, effectuant de cette manière une modification souhaitée dans le phénotype des cellules et induisant ou amplifiant l'expression de l'ABCG2 dans les mêmes cellules,
- sélectionner les cellules exprimant l'ABCG2 et présentant le phénotype modifié par un médicament cytotoxique pouvant être transporté par l'ABCG2,
- différencier les cellules en cellules immatures ou matures et
- introduire les cellules différenciées chez un patient,
où de préférence le sujet à partir duquel les cellules sont isolées est identique au patient traité.

9. Combinaison ou kit pour une utilisation selon les revendications 7 ou 8, où les cellules isolées à traiter sont des cellules présentant l'antigène (CPA) ou des cellules précurseurs d'une CPA, de préférence des monocytes, de manière davantage préférée des cellules dendritiques (CD) et
le PPARg et, le cas échéant, le RXR sont activés dans lesdites cellules par l'administration aux cellules d'un agoniste du PPARg et, le cas échéant, d'un agoniste du RXR.

10. Procédé *in vitro* de protection des cellules mammaliennes somatiques contre l'effet d'un médicament cytotoxique pouvant être transporté par ABCG2, comprenant les étapes consistant à
- fournir lesdites cellules sous une forme isolée,
- amplifier l'expression de ABCG2 par l'activation ou l'augmentation de l'expression du PPARg et, le cas échéant, du RXR dans lesdites cellules,
- exposer les cellules à l'effet d'un médicament cytotoxique pouvant être transporté par l'ABCG2.

11. Procédé selon la revendication 10, dans lequel le PPARg est activé par l'administration aux cellules d'un agoniste du PPARg, et éventuellement, le RXR est activé par l'administration aux cellules d'un agoniste du RXR.

12. Procédé selon la revendication 10, dans lequel l'expression du PPARg et, le cas échéant, du RXR est effectuée par un transfert génique dans les cellules thérapeutiques.

13. Agoniste du PPARg pour une utilisation visant à conférer une cytoprotection aux cellules immunitaires mammaliennes contre un médicament cytotoxique pouvant être transporté par l'ABCG2.


### SEQUENCE LISTING

<110> University of Debrecen
   Nagy, Laszlo
   Szatmari, Istvan
<120> Compounds, kits and methods for conferring cytoprotection
<130> P103056/SG
<150> P0600997
<151> 2006-06-14
<150> P0600508
<151> 2006-06-19
<160> 16
   <170> PatentIn version 3.3
<210> 1
   <211> 13
   <212> DNA
   <213> Homo sapiens
<400> 1
   agggcagagg gca 13
<210> 2
   <211> 13
   <212> DNA
   <213> Homo sapiens
<400> 2
   taacctttaa cct 13
<210> 3
   <211> 13
   <212> DNA
   <213> Homo sapiens
<400> 3
   taacatctga cct 13
<210> 4
   <211> 13
   <212> DNA
   <213> Canis familiaris
<400> 4
   taacttttga cct 13
<210> 5
   <211> 13
   <212> DNA
   <213> Homo sapiens
<400> 5
   taacttttaa cct 13
<210> 6
   <211> 150
   <212> DNA
   <213> Homo sapiens
<400> 6
   gtggtggagg gcagagggca atggcttgga aggctgtgag tcacttaacc tttaaccttg 60
   tgcaaatcag tctcctttgc actggtgact tatctgtaaa atgaggaaag tcatctaaca 120
   tctgacctaa tgaggaaggt cctttttctc 150
<210> 7
   <211> 211
   <212> DNA
   <213> Canis familiaris
<400> 7
   ccctctaact tttgaccttg tcatactggt ctcctttgta ccgggtgact catctgtaac 60
   gttatgacac agaggacaga gagcagcagt ctcagaagct gtgagttccc ctatgcacaa 120
   ttagcaaggt catctttaga ttcctccctc actatattgt gacctgaaac atccaaacac 180
   taaaagacag gacaggacat ttgaaaatga t 211
<210> 8
   <211> 231
   <212> DNA
   <213> Bos taurus
<400> 8
   cctcctaact tttgaccttg tgcaagatgg cctcatttgg accaggtggg tcatcttaac 60
   tgttcaaaga gggtgagcag agggcagtgg tctgagaaac tgagtcaccc atttataatt 120
   aggaaggtag tctttaggtt cctccctccc taatattcta ttacctaaaa tacccaaaca 180
   ctaaatgaga aaacagggca tttgaaaata gttggttttg ttcagtcact a 231
<210> 9
   <211> 13
   <212> DNA
   <213> Homo sapiens
<220>
   <221> misc_feature
<222> (7)..(7)
   <223> n is a, c, g, or t
<400> 9
   aggtcanagg tca 13
<210> 10
   <211> 21
   <212> DNA
   <213> Artificial
<220>
   <223> PPARg specific siRNA sequence for RNA interference studies
<220>
   <221> misc_feature
   <222> (20)..(20)
   <223> deoxy thymine
<220>
   <221> misc_feature
   <222> (21)..(21)
   <223> deoxy thymine
<400> 10
   gcccttcact actgttgact t 21
<210> 11
   <211> 25
   <212> DNA
   <213> Artificial
<220>
   <223> oligonucleotide for mobility shift assay
<400> 11
   ggggtggagg gcagagggca atggc 25
<210> 12
   <211> 25
   <212> DNA
   <213> Artificial
<220>
   <223> oligonucleotide for mobility shift assay
<400> 12
   ggggtgggcg agagagggca atggc 25
<210> 13
   <211> 25
   <212> DNA
   <213> Artificial
   <220>
   <223> oligonucleotide for mobility shift assay
<400> 13
   gggcacaagg ttaaaggtta agtga 25
<210> 14
   <211> 25
   <212> DNA
   <213> Artificial
<220>
   <223> oligonucleotide for mobility shift assay
<400> 14
   gggcacattg agaaaggtta agtga 25
<210> 15
   <211> 25
   <212> DNA
   <213> Artificial
<220>
   <223> oligonucleotide for mobility shift assay
<400> 15
   ggtcattagg tcagatgtta gatga 25
<210> 16
   <211> 25
   <212> DNA
   <213> Artificial
<220>
   <223> oligonucleotide for mobility shift assay
<400> 16
   ggtcatttcg agagatgtta gatga 25